# EUROPÄISCHE PATENTSCHRIFT

(11) **EP 1 187 846 B1**
(45) Veröffentlichungstag und Bekanntmachung des Hinweises auf die Patenterteilung: **18.04.2007**
(21) Anmeldenummer: 00947882.7
(22) Anmeldetag: 26.06.2000
(51) Int. Cl.: C07J 41/00, C07J 43/00, A61K 31/565, A61K 31/58, A61P 5/32, A61P 5/30

(54) **11BETA-LANGKETTIG-SUBSTITUIERTE ESTRATRIENE, VERFAHREN ZUR HERSTELLUNG, PHARMAZEUTISCHE PRÄPARATE, DIE DIESE 11BETA-LANGKETTIG-SUBSTITUIERTEN ESTRATRIENE ENTHALTEN, SOWIE DEREN VERWENDUNG ZUR HERSTELLUNG VON ARZNEIMITTELN**
11BETA LONG-CHAIN SUBSTITUTED ESTRATRIENES, METHOD FOR THEIR PRODUCTION, PHARMACEUTICAL PREPARATIONS CONTAINING SAID 11BETA LONG-CHAIN SUBSTITUTED ESTRATRIENES, AND THEIR USE FOR PRODUCING MEDICAMENTS
OESTRATRIENES 11BETA A CHAINE LONGUE SUBSTITUES, PROCEDE DE PREPARATION, PREPARATIONS PHARMACEUTIQUES CONTENANT CES OESTRATRIENES, AINSI QUE LEUR UTILISATION POUR LA FABRICATION DE MEDICAMENTS

(30) Priorität: 24.06.1999 DE 19929715
(43) Veröffentlichungstag der Anmeldung: 20.03.2002
(73) Patentinhaber: Bayer Schering Pharma AG, 13353 Berlin (DE)
(72) Erfinder: BOHLMANN, Rolf, D-14055 Berlin (DE); HEINRICH, Nikolaus, D-12159 Berlin (DE); KROLL, Jorg, D-12157 Berlin (DE); SAUER, Gerhard, D-13465 Berlin (DE); FRITZEMEIER, Karl-Heinrich, D-13503 Berlin (DE); HEGELE-HARTUNG, Christa, D-45470 Mülheim a.d. Ruhr (DE); HOFFMANN, Jens, D-16567 Mühlenbeck (DE); LICHTNER, Rosemarie, D-10823 Berlin (DE); ZORN, Ludwig, D-13509 Berlin (DE)
(86) Internationale Anmeldenummer: PCT/EP2000/005969
(87) Internationale Veröffentlichungsnummer: WO 2001/000652

(56) Entgegenhaltungen:
- EP-A- 0 384 842
- EP-A- 0 850 647
- WO-A-00/31112
- WO-A-93/13123
- WO-A-97/30697
- WO-A-98/28324
- WO-A-99/25725
- FR-A- 2 640 977
- C LOBACCARO ET AL: "Steroidal Affinity Labels of the Estrogen Receptor. 3. Estradiol 11.beta.-n-Alkyl Derivatives Bearing a Terminal Electrophilic Group: Anti-estrogenic and Cytotoxic Properties" JOURNAL OF MEDICINAL CHEMISTRY,US,AMERICAN CHEMICAL SOCIETY. WASHINGTON, Bd. 40, Nr. 14, 4. Juli 1997 (1997-07-04), Seiten 2217-2227, XP002100729 ISSN: 0022-2623

## Beschreibung

Die vorliegende Erfindung betrifft 11β-langkettig-substituierte Estratriene der allgemeinen Formel I 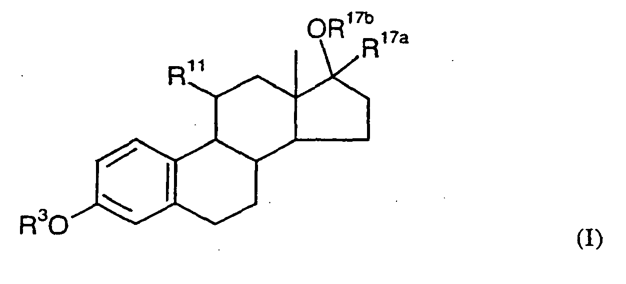 worin
R³ ein Wasserstoffatom, einen Kohlenwasserstoffrest mit bis zu 8 Kohlenstoffatomen oder einen Rest der Teilformel R^{3'}-C(O)-, worin R^{3'} ein Wasserstoffatom oder ein Kohlenwasserstoffrest mit bis zu 8 Kohlenstoffatomen oder ein Phenylrest ist, bedeutet,
R¹¹ einen Rest der Formel -A-B-Z-R²⁰ bedeutet,
   worin
   A für eine direkte Bindung und
   B für eine gerad- oder verzweigtkettige Alkylen-, Alkenylen- oder Alkinylengruppe mit 4, 5 oder 6 Kohlenstoffatomen
   stehen, und
   Z für -NR²¹- und R²¹ für eine C₁-C₃-Alkylgruppe stehen,
      wobei R²⁰
      ein Wasserstoffatom,
      eine gerad- oder verzweigtkettige Alkyl-, Alkenyl- oder Alkinylgruppe mit bis zu 10 Kohlenstoffatomen, wobei, R²⁰ und R²¹ aber beide nicht gleichzeitig Methyl, sein dürfen,
      oder eine der Gruppierungen
      -D-CₙF₂ₙ₊₁, wobei D eine gerad- oder verzweigtkettige Alkylen-, Alkenylen- oder Alkinylengruppe mit bis zu 8 Kohlenstoffatomen und n eine ganze Zahl von 1 bis 8 ist,
      D-Aryl, wobei D die bereits angegebene Bedeutung hat und Aryl für einen gegebenenfalls ein- oder zweifach substituierten Phenyl-, 1- oder 2-Naphthyl- oder einen Heteroarylrest steht,
      -L-CH=CF-CₚF₂ₚ₊₁, wobei L eine gerad- oder verzweigtkettige Alkylen-, Alkenylen- oder Alkinylengruppe mi bis zu 7 Kohlenstoffatomen und p eine ganze Zahl von 1 bis 7 ist,
      wobei in den drei vorstehenden Fällen in D bzw. L eine Methylengruppe durch ein Schwefelatom, eine Sulfon- oder Sulfoxidgruppe ersetzt sein kann,
      -D-O-(CH₂)_{q}-Aryl, wobei D und Aryl die bereits angegebenen Bedeutungen haben und q 0, 1,2 oder 3 ist,
      -D-O-(CH₂)ᵣ-CₙF₂ₙ₊₁, wobei D und n die bereits angegebenen Bedeutungen haben und r für eine ganze Zahl von 1 bis 5 steht,
      bedeutet,
      wobei außerdem in allen betreffenden vorstehenden Fällen R²¹ gemeinsam mit D unter Einschluß des Stickstoffatoms einen dann in 2- oder 3-Stellung substituierten Pyrrolidinring bilden kann,
      oder
   R²⁰ und R²¹ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält und gegebenenfalls substituiert ist,
      bilden,
      und
R^{17a} in α- oder β-Position ein Wasserstoffatom, eine -C₁₋₅-Alkyl-, eine C₂₋₅-Alkenyl- oder eine C₂₋₅-Alkinylgruppe oder eine Trifluormethylgruppe
   oder gemeinsam mit dem Rest OR^{17b} ein Ketosauerstoffatom bedeutet, und
R^{17b} ein Wasserstoffatom oder einen Rest der Teilformel R^{17'}-C(O)-, worin R^{17'} ein Wasserstoffatom oder ein Kohlenwasserstoffrest mit bis zu 8 Kohlenstoffatomen ist, bedeutet.

Die erfindungsgemäßen substituierte-Estratriene weisen als R³ vorzugsweise ein Wasserstoffatom auf. Die Hydroxygruppe kann aber auch mit einem gerad- oder verzweigtkettigen, gesättigten oder ungesättigten Kohlenwasserstoffrest mit bis zu 8 Kohlenstoffatomen, wie z.B. einer Methyl-, Ethyl-, Propyl-, Isopropyl-, Butyl-, Isobutyl-, tert.-Butyl-, Pentyl,- Isopentyl-, Neopentyl-, Heptyl-, Hexyl- oder Octylrest verethert oder mit einem Acylrest R^{3'}-C(O)-, worin R^{3'} ein Wasserstoffatom oder ein Kohlenwasserstoffrest mit bis zu 8 Kohlenstoffatomen oder ein Phenylrest ist, verestert sein.

Für den Substituenten R^{17b} kann ein Wasserstoffatom oder einen Rest der Teilformel R^{17'}-C(O)- stehen, worin R^{17'} ein Wasserstoffatom oder ein Kohlenwasserstoffrest mit bis zu 8 Kohlenstoffatomen ist. Ein Wasserstoffatom ist für R^{17b} bevorzugt. Der Kohlenwasserstoffrest kann beispielsweise die Bedeutung eines Methyl-, Ethyl-, Propyl-, Isopropyl-, Butyl-, Isobutyl-, tert.-Butyl-, Pentyl,- Isopentyl-, Neopentyl-, Heptyl-, Hexyl- oder Octylrestes haben. Weiterhin kann der Substituent -OR^{17b} α- oder β-ständig sein. Die β-Position ist bevorzugt.

R^{17a} kann ein Wasserstoffatom, einen geradkettigen oder verzweigten C₁₋₅-Alkylrest, wie beispielsweise einen Methyl-, Ethyl-, Propyl-, Isopropyl-, Butyl-, Isobutyl-, tert.-Butyl-, Pentyl,- Isopentyl-, Neopentylrest, einen geradkettigen oder verzweigten C₂₋₅-Alkenylrest wie beispielsweise einen Ethenyl-, 1-Propenyl, 2-Propenyl, Isopropenyl, 1-Butenyl, 2-Butenyl-, 3-Butenyl, 1-Ethyl-ethenyl, 2-Ethylethenyl. 1-Methyl(1-Propenyl). 1-Methyl(2-propenyl)rest oder einen geradkettigen oder verzweigten C₂₋₅-Alkinylrest wie beispielsweise einen Ethinyl-, 1-Popinyl-, 2-Propinyl-, 1-Butinyl-, 2-Butinyl-, 3-Butinyl-. 3-Methyl(1-Butinyl)-, 1-Methyl(3-Butinyl)rest und einen Trifluormethylrest bedeuten.
Bevorzugt bedeutet R^{17b} ein Wasserstoffatom, eine, C₂₋₃-Alkenyl- einen C₂₋₃ Alkinylrest oder eine Trifluormethylgruppe.
Besonders bevorzugt bedeutet R^{17a} ein Wasserstoffatom, eine Methylgruppe, einen Ethenylrest, einen Ethinylrest oder eine Trifluormethylgruppe.
Weiterhin kann der Rest R^{17a} α- oder β-ständig sein. Die α-Position ist für R^{17a} bevorzugt.

Eine weitere Bedeutung für R^{17b} gemeinsam mit OR^{17a} ist eine Ketosauerstofatom. Diese Bedeutung soll vor jeder anderen Substitution in der 17-Position bevorzugt sein.

In den erfindungsgemäßen Verbindungen der allgemeinen Formel I steht A für eine direkte Bindung.

Beim einem Arylrest, der gegebenenfalls substituiert sein kann, handelt es sich im Sinne der vorliegenden Erfindung um einen Phenyl-, 1- oder 2-Naphthylrest; der Phenylrest ist bevorzugt. Wenn nicht ausdrücklich erwähnt, schließt Aryl immer auch einen Heteroarylrest mit ein. Beispiele für einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3- oder 4-Pyridazinylrest.

Wenn R²⁰ und R²¹ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält, ist dies insbesondere ein Pyrrolidin-, Piperidin-, Morpholin- oder Piperazinring.

Als Substituenten für den Aryl-, Heteroaryl-, Aralkyl- und Heteroarylalkylrest seien beispielsweise ein Methyl-, Ethyl-, Propyl-, Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind (Aralkyl siehe oben bei R²⁰ und R³¹) oder der 1-Methoxyacetylaminorest genannt.

Das Schwefelatom in der Seitenkette kann als einfache Schwefelbrücke (Sulfid), als Sulfon oder Sulfoxid vorliegen.

Als spezifische Seitenketten seien genannt

-(CH₂)₅N(CH₃)-(CH₂)₃-S-(CH₂)₃C₂F₅

-(CH₂)₅NH-(CH₂)₃-S-(CH₂)₃C₂F₅

-(CH₂)₅N(CH₃)-(CH₂)₃-S-CH₂-2-Pyridyl

-(CH₂)₅N(CH₃)-(CH₂)₃-SO-CH₂-2-Pyridyl

-(CH₂)₅N(CH₃)-(CH₂)₃-S-CH₂-p-CF₃-Phenyl

-(CH₂)₅N(CH₃)-(CH₂)₃-SO-CH₂-p-CF₃-Phenyl

-(CH₂)₅-[2-Pyrrolidin-1-yl]-CH₂-S-p-CF₃-Phenyl

-(CH₂)₅-[2-Pyrrolidin-1-yl]-CH₂-SO-p-CF₃-Phenyl

-(CH₂)₅N(CH₃)(CH₂)₃C₂F₅

-(CH₂)₅N(CH₃)(CH₂)₆C₂F₅

-(CH₂)₅N(CH₃)(CH₂)₇C₂F₅

-(CH₂)₅N(CH₃)(CH₂)₈C₂F₅

-(CH₂)₆N(CH₃)(CH₂)₆C₂F₅

-(CH₂)₆N(CH₃)(CH₂)₇C₂F₅

-(CH₂)₆N(CH₃)(CH₂)₈C₂F₅

-(CH₂)₅N(CH₃)(CH₂)₂C₄F₉

-(CH₂)₅N(CH₃)(CH₂)₃C₆F₁₃

-(CH₂)₅N(CH₃)(CH₂)₃C₈F₁₇

-(CH₂)₅N(CH₃)(CH₂)₆C₄F₉

-(CH₂)₅N(CH₃)(CH₂)₆C₆F₁₃

-(CH₂)₅N(CH₃)(CH₂)₆C₈F₁₇

-(CH₂)₅N(CH₃)H

-(CH₂)₅N(CH₃)(CH₂)₉H

-(CH₂)₅N(CH₃)CH₂CH=CF-C₂F₅

-(CH₂)₅N(CH₃)CH₂CH=CF-C₃F₇

-(CH₂)₅N(CH₃)CH₂CH=CF-C₅F₁₇

-(CH₂)₅N(CH₃)CH₂CH=CF-C₇F₁₅

-(CH₂)₅-1-Pyrrolidinyl

-(CH₂)₅N(CH₃)(CH₂)₃OPhenyl

-(CH₂)₅N(CH₃)(CH₂)₃OBenzyl

-(CH₂)₅N(CH₃)(CH₂)₃O(CH₂)₃C₂F₅

-(CH₂)₅N(CH₃)(CH₂)₃CH(CH₃)₂

-(CH₂)₅N(CH₃)(CH₂)₃-Pyridyl

-(CH₂)₅N(CH₃)(CH₂)₃-Phenyl

-(CH₂)₅N(CH₃)(CH₂)₂-p-Tolyl

-(CH₂)₅N(CH₃)(CH₂)₂-p-Ethoxyphenyl

-(CH₂)₅N(CH₃)(CH₂)₃-p-Tolyl

-(CH₂)₅N(CH₃)(CH₂)₃-p-Chlorphenyl

-(CH₂)₅N(CH₃)(CH₂)₃-O-CH₂-Phenyl

-(CH₂)₅N(CH₃)(CH₂)₂-O-p-Br-Phenyl

-(CH₂)₅N(CH₃)(CH₂)₂-O-p-CF₃-Phenyl

Die vorliegende Erfindung betrifft unter anderen die folgenden Verbindungen:
11β-[5-(Methyl{3-[(4,4,5,5,5-pentafluorpentyl)sulfanyl]propyl}amino)pentyl]estra-1,3,5(10)-trien-3,17β-diol
11β-(5-{3-[(4,4,5,5,5-Pentafluorpentyl)sulfanyl]propylamino}pentyl)estra-1,3,5(10)-trien-3,17β-diol
11β-[5-(Methyl{3-[(2-pyridylmethyl)sulfanyl]propyl}amino)pentyl]estra-1,3,5(10)-trien-3,17β-diol
11β-[5-(Methyl{3-[(2-pyridylmethyl)sulfinyl]propyl}amino)pentyl]estra-1,3,5(10)-trien-3,17β-diol
11β-[5-(Methyl{3-[4-(trifluormethyl)benzylsulfanyl]propyl}amino)pentyl]estra-1,3,5(10)-trien-3,17β-diol
11β-[5-(Methyl(3-[4-(trifluormethyl)benzylsulfinyl]propyl)amino)pentyl]estra-1,3,5(10)-trien-3,17β-diol
11β-{5-[(2S)-2-{[4-(Trifluormethyl)phenyl]sulfanylmethyl}pyrrolidin-1-yl]pentyl}estra-1,3,5(10)-trien-3,17β-diol
11β-{5-[(2S)-2-{[4-(Trifluormethyl)phenyl]sulfinylmethyl}pyrrolidin-1-yl]pentyl}estra-1,3,5(10)-trien-3,17β-diol
11β-{5-[methyl-(8,8,9,9,9-pentafluor-nonyl)-amino]-pentyl}-estra-1,3,5(10)-trien-3,17β-diol
11β-{5-[methyl-nonyl-amino]-pentyl}-estra-1,3,5(10)-trien-3,17β-diol
11β-{5-[methyl-(9,9,10,10,10-pentafluor-decyl)-amino]-pentyl}-estra-1,3,5(10)-trien-3,17β-diol
11β-{6-[methyl-(8,8,9,9,9-pentafluor-nonyl)-amino]-hexyl}-estra-1,3,5(10)-trien-3,17β-diol
11β-{6-[methyl-(9,9,10,10,10-pentafluor-decyl)-amino]-hexyl}-estra-1,3,5(10)-trien-3,17β-diol
11β-(5-(methyl-amino)-pentyl)-estra-1,3,5(10)-trien-3,17β-diol
11β-(5-pyrrolidin-1-yl-pentyl)-estra-1,3,5(10)-trien-3,17β-diol
11β-{5-[methyl-(4,4,5,5,5-pentafluor-pentyl)-amino]-pentyl}-estra-1,3,5(10)-trien-3,17β-diol
11β-{5-[methyl-(4,4,5,5,6,6,7,7,8,8,9,9,9-tridecafluor-nonyl)-amino]-pentyl}-estra-1,3,5(10)-trien-3,17β-diol
11β-{5-[(4,4,5,5,6,6,7,7,8,8,9,9,10,10,11,11,11-heptadecafluor-undecyl)-methyl-amino]-pentyl}-estra-1,3,5(10)-trien-3,17β-diol
11β-{5-[methyl-(3,3,4,4,5,5,6,6,6-nonafluor-hexyl)-amino]-pentyl}-estra-1,3,5(10)-trien-3,17 β-diol
11β-{(5-[methyl-(7,7,8,8,8-pentafluor-octyl)-amino]-pentyl)-estra-1,3,5(10)-trien-3,17β-diol
11β-{6-[methyl-(7,7,8,8,8-pentafluor-octyl)-amino]-hexyl}-estra-1,3,5(10)-trien-3,17β-diol
11β-{5-[methyl-(7,7,8,8,9,9,10,10,10-nonafluor-decyl)-amino]-pentyl}-estra-1,3,5(10)-trien-3,17β-diol
11β-{5-[methyl-(7,7,8,8,9,9,10,10,11,11,12,12,12-tridecafluor-dodecyl)-amino]-pentyl}-estra-1,3,5(10)-trien-3,17β-diol
11β-{5-[(7,7,8,8,9,9,10,10,11,11,12,12,13, 13,14.14,14-heptadecafluor-tetradecyl)-methyl-amino]-pentyl}-estra-1,3,5(10)-trien-3,17β-diol
11β-{5-[(3,4,4,5,5,5-hexafluor-pent-2-enyl)-methyl-amino]-pentyl}-estra-1,3,5(10)-trien-3,17 β-diol
11β-{5-[(3,4,4,5,5,6,6,7,7,8,8,8-Dodecafluor-oct-2-enyl)-methyl-amino]-pentyl}-estra-1,3,5(10)-trien-3,17β-diol
11β-{5-[(3,4,4,5,5,6,6,7,7,8,8,9,9,10,10,10-hexadecafluor-dec-2-enyl)-methyl-amino}-pentyl}-estra-1,3,5(10)-trien-3,17β-diol
11β-{5-methyl-(3-phenoxy-propyl)-amino]-pentyl}-estra-1,3,5(10)-trien-3,17β-diol
11β-{5-[(3-Benzyloxy-propyl)-methyl-amino)-pentyl}-estra-1,3,5(10)-trien-3,17β-diol
11β-{5-[N-methyl-N-3-(4,4,5,5,5-pentafluorpentyloxy)-propylamino]-pentyl}-estra-1,3,5(10)-trien-3,17β-diol
11β-{5-[methyl-(2-p-tolyl-ethyl)-amino]-pentyl}-estra-1,3,5(10)-trien-3,17β-diol
11β-(5-{[2-(4-Ethoxy-phenyl)-ethyl)-methyl-amino}-pentyl)-estra-1,3,5(10)-trien-3,17β-diol
11β-{5-[methyl-(3-phenyl-propyl)-amino]-pentyl}-estra-1,3,5(10)-trien-3,17β-diol
11β-{5-[methyl-(3-pyridin-3-yl-propyl)-amino]-pentyl}-estra-1,3,5(10)-trien-3,17β-diol
11β-{5-[methyl-(3-p-tolyl-propyl)-amino)-pentyl}-estra-1,3,5(10)-trien-3,17β-diol
11β-(5-{[3-(4-Chlor-phenyl)-propyl]-methyl-amino}-pentyl)-estra-1,3,5(10)-trien-3,17β-diol
11β-(5-{([3-(4-Ethoxy-phenyl)-propyl]-methyl-amino}-pentyl)-estra-1,3,5(10)-trien-3,17β-diol
11β-{5-[methyl-(4-methyl-pentyl)-amino]-pentyl}-estra-1,3,5(10)-trien-3,17β-diol

Die vorliegende Erfindung betrifft außer diesen Verbindungen der allgemeinen Formel I auch deren physiologisch verträgliche Additionssalze mit organischen und anorganischen Säuren, diese Verbindungen der allgemeinen Formel I inclusive der Additionssalze enthaltende pharmazeutische Präparate sowie deren Verwendung zur Herstellung von Arzneimitteln.

Zur Bildung von Säureadditionssalzen sind anorganische und organische Säuren geeignet, wie sie dem Fachmann zur Bildung physiologisch verträglicher Salze bekannt sind. Als Additionssalze mit Säuren sind insbesondere die Hydrochloride, Hydrobromide, Acetate, Citrate, Oxalate, Tartrate und die Methansulfonate zu nennen.

Die Verbindungen der allgemeinen Formel I stellen Verbindungen mit starker antiestrogener Wirksamkeit und mit überraschend oraler Verwendungsmöglichkeit dar.

Bei den erfmdungsgemäßen Verbindungen handelt es sich zum einen Teil um reine Antiestrogene oder zum anderen Teil um sogenannte Partialantagonisten, d. h. um Antiestrogene mit estrogener Partialwirkung wie das Tamoxifen oder das Raloxifen. Im Gegensatz zum Tamoxifen tritt bei den Partialantagonisten der allgemeinen Formel I deren agonistische, estrogene Wirkung gewebeselektiv auf. Insbesondere tritt die agonistische Wirkung am Knochen, im Herz-Kreislaufsystem und im ZNS (Zentrales Nervensystem) auf. Insbesondere tritt am Uterus keine oder nur geringe agonistische Wirkung tritt auf.

Verbindungen mit antiestrogenen Eigenschaften, d.h. Stoffe mit Hemmwirkungen gegenüber. Estrogenen, sind bereits zahlreich beschrieben worden.

In 11-Stellung einen β-ständigen Substituenten tragende Estratriene, die unter anderem auch antiestrogene Wirkung aufweisen, gehen beispielsweise aus folgenden Patentanmeldungen bzw. Patenten hervor:
WO 98/28324, EP-A 0 850 647 , EP-A 0 629 635, WO 93/13123, EP-A 0 558 416, EP-A 0 471 612, EP-A 0 384 842, EP-B 0 097 572, WO/99 25725.

Weiterhin sind die aus der EP 0 138 504 B 1 hervorgehenden Steroid-Derivatezu erwähnen. Das 7α-[9-(4,4,5,5,5-Pentafluorpentylsulfinyl)-n-nonyl)-estra-1,3,5(10)-trien-3,17β-diol befindet sich gegenwärtig in klinischer Entwicklung für hormonabhängige Tumoren (Brustkrebs).

Pharmazeutische Zusammensetzungen, die Sexualsteroid-Inhibitoren enthalten, welche ein steroidales Grundgerüst, das eine 7α-Seitenkette bei gleichzeitiger Anwesenheit mindestens eines weiteren Substituenten in Position 14, 15 oder 16 aufweist, sind Gegenstand der EP-A 0 376 576.

Antiestrogen wirksame Estratriene, die ein 11β-Fluoratom tragen können und in Position 7 eine α-ständige Seitenkette tragen, die eine Amino- und eine Schwefelfunktion aufweist und die endständig funktionalisiert ist, sind in der WO 98/07740 beschrieben.

Bei den erfindungsgemäßen Verbindungen handelt es sich um Verbindungen mit starker antiestrogener Wirkung nach peroraler Applikation.

Der Antiuteruswachstumstest bei der infantilen Ratte, s.c. und p.o. (Test auf antiestrogene Wirkung in-vivo) belegt die antiestrogene Wirksamkeit der erfindungsgemäßen Verbindungen. Der Test wird wie nachstehend beschrieben durchgeführt:

### Uteruswachstumstest bei der infantilen Ratte (antiestrogene Wirkung)

### Prinzip der Methode

Bei Nagern reagiert der Uterus auf die Applikation von estrogenen mit einer Gewichtszunahme (sowohl Proliferation als auch Wassereinlagerung). Dieses Wachstum ist durch gleichzeitige Gabe antiestrogen-wirkender Verbindungen dosisabhängig zu hemmen.

### Versuchsdurchführung

### Tiere:

Infantile weibliche Ratten im Gewicht von 35-45 g bei Versuchsbeginn, pro Dosis 5-6 Tiere.

### Formulierung und Applikation der Substanzen:

Für die p.o. Applikation werden die Substanzen in 1 Teil Ethanol (E) gelöst und mit 9 Teilen Erdnußöl (EÖ) aufgefüllt.

### Versuchsansatz

Die gerade von den Müttern abgesetzten jungen Ratten werden zur Eingewöhnung einen Tag vor Behandlungsbeginn geliefert und sofort mit Futter - auch in dem Tierkäfig - versorgt. Die Behandlung erfolgt dann täglich einmal über 3 Tage in Kombination mit 0,5 µg Estradiolbenzoat (EB). EB wird immer subcutan (s.c.) appliziert, während die Testsubstanz p.o. (peroral) verabreicht wird. 24 Stunden nach der letzten Applikation werden die Tiere gewogen, getötet und die Uteri entnommen. Von den präparierten Uteri werden die Feuchtgewichte (ohne Inhalt) ermittelt.

### Kontrollen

negative Kontrolle: Vehikel (E/EÖ), 0,2 ml/Tier/Tag
positive Kontrolle: 0,5 µg EB/0,1 ml/Tier/Tag

### Auswertung

Von den relativen Organgewichten (mg/100 g Körpergewicht) werden für jede Gruppe die Mittelwerte mit Standardabweichung (X+SD), sowie die Signifikanz der Unterschiede zur Kontrollgruppe (EB) im Dunnett-Test (p<0.05) ermittelt. Die Berechnung der Hemmung (in %) gegenüber der EB-Kontrolle erfolgt mit einem Programm. Die relativen Wirksamkeiten der Prüfsubstanzen werden durch eine Kovarianz- und Regressionsanalyse ermittelt.

Als reine Antiestrogene im Sinne vorliegender Erfindung sind solche Verbindungen der allgemeinen Formel I anzusehen, die im in-vivo Test auf estrogene Wirkung keine oder bestenfalls nur geringfügige agonistische Wirkung zeigen.

Mittels der nachstehend beschriebenen Methode läßt sich der estrogene Effekt erfindungsgemäßer Verbindungen auf den Knochen ermitteln. Bei selektiv estrogen wirksamen Verbindungen werden bei vergleichbaren Dosierungen am Knochen protektive Effekte beobachtet, während am Uterus keine oder bestenfalls eine geringfügige Stimulierung festgestellt wird.

### Knochenuntersuchungen

### Methode

3 Monate alte weibliche Ratten werden ovarektomiert und unmittelbar nach der Operation 28 Tage lang 1mal täglich mit der Testverbindung behandelt. Die Applikation erfolgt subcutan in Rizinusöl/Benzylbenzoat oder Arachisöl/Ethanol. Die Tiere werden am Tag nach der letzten Applikation getötet und Femur, Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 5 - 7 mm vom Gelenkkopf am distalen Femur oder der proximalen Tibia durchgeführt.

Alternativ wird die Wirkung am Knochen durch Ausmessen der trabekulären Knochenfläche der sekundären Spongiosa an histologischen Präparaten des distalen Femur oder der proximalen Tibia festgestellt. Das Ergebnis wird als prozentualer Anteil der trabekulären Knochenfläche an der ausgemessenen Gesamtknochenfläche ausgedrückt (TB/BV). Die mittels QCT gemessene Knochendichte und die am histologischen Schnitt ermittelte trabekuläre Knochenfläche korrelieren gut miteinander. Ein Vergleich beider Messgrößen ist deshalb zulässig.

Der Übergang zwischen den reinen Antiestrogenen und den Partialagonisten, den gewebeselektiven Estrogenen, ist fließend. Verbindungen, die eine geringfügige agonistische Wirkung aufweisen, können ebenso in den nachfolgend für die reinen Antiestrogene genannten Indikationen verwendet werden

Die erfindungsgemäßen Verbindungen, insbesondere wenn es reine Antiestrogene sind, eignen sich zur Therapie von estrogen-abhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Prostatacarcinom, Prostatahyperplasie, anovulatorische Infertilität und Melanom.
Die reinen Antiestrogene der allgemeinen Formel I können außerdem als Komponente in den in der EP 346 014 B 1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen. Die Verbindungen der allgemeinen Formel I, insbesondere wenn es sich um reine Antiestrogene handelt, können gemeinsam mit Antigestagenen (kompetitiven Progesteronantagonisten) zur Behandlung hormonabhängiger Tumoren verwendet werden (EP 310 542 A).
Weitere Indikationen, in denen die Verbindungen der allgemeinen Formel zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530).

Außerdem können die Verbindungen der allgemeinen Formel I zur Herstellung von Medikamenten zur Behandlung der Endometriose und von Endometrialkarzinomen verwendet werden.
Ferner kann man die Verbindungen der allgemeinen Formel I zur Herstellung pharmazeutischer Zusammensetzungen für die männliche und weibliche Fertilitätskontrolle einsetzen (männliche Fertilitätskontrolle: DE-A 195 10 862.0).

Diejenigen Verbindungen der allgemeinen Formel I mit gewebeselektiver estrogener Partialwirkung können in erster Linie zur Prophylaxe und Therapie der Osteoporose und zur Herstellung von Präparaten für die Substitutionstherapie in der Prae-, Peri- und Postmenopause (HRT = Hormone Replacement Therapy; Hormonersatz-Therapie) Verwendung finden (Black, L.J., Sato,M.,Rowley, E.R.,Magee, D.E., Bekele, A., Williams, D.C., Cullinan, G.J., Bendele, R., Kauffman, R.F., Bensch, W.R., Frolik, C.A., Termine, J.D. and Bryant, H.U.: Raloxifene [LY 139481 HCl] prevents bone loss and reduces serum cholesterol without causing uterine hypertrophy in ovariectomized rats; J. Clin. Invest. 93: 63 - 69, 1994).

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere zur Behandlung von estrogenabhängigen Krankheiten und Tumoren und von Arzneimitteln für die Hormonsubstitutions-Therapie (HRT).

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind. Heft 2, 1961, Seite 72 u. ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zu verabreichende Menge der Verbindungen schwankt innerhalb eines weiten Bereichs und kann jede wirksame Menge abdecken. In Abhängigkeit des zu behandelnden Zustandes und der Art der Verabreichung kann die Menge der verabreichten Verbindung 0,1-25 mg/kg Körpergewicht, vorzugsweise 0,5-5 mg/kg Körpergewicht, je Tag betragen. Beim Menschen entspricht dies einer täglichen Dosis von 5 bis 1250 mg. Die bevorzugte tägliche Dosierung beim Menschen ist 50 bis 200 mg. Dies gilt insbesondere für die Tumortherapie.

Zur oralen Verabreichung kommen Kapseln, Pillen, Tabletten, Dragees usw. infrage. die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw., enthalten. Die einzelnen Dosierungseinheiten für die orale Applikation können beispielsweise 5 bis 500 mg des Wirkstoffs enthalten.

Um eine bessere Bioverfügbarkeit des Wirkstoffes zu erreichen, können die Verbindungen auch als Cyclodextrinclathrate formuliert werden. Hierzu werden die Verbindungen mit α-, β-oder γ-Cyclodextrin oder Derivaten von diesen umgesetzt (PCT/EP95/02656).

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen der allgemeinen Formel I können auch in Form einer Lösung formuliert werden, die für die orale Verabreichung bestimmt ist und die neben der aktiven Verbindung der allgemeinen Formel I enthält
a) ein pharmazeutisch verträgliches Öl und/oder
b) eine pharmazeutisch verträgliche lipophile oberflächenaktive Substanz und/oder
c) eine pharmazeutisch verträgliche hydrophile oberfächenaktive Substanz und/oder
d) ein pharmazeutisch verträgliches wassermischbares Lösungsmittel.
Hierzu wird außerdem auf die WO 97/21440 verwiesen.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen) eignen sich verschiedene Polymere wie zum Beispiel Silikonpolymere, Ethylenvinylacetat, Polyethylen oder Polypropylen.

Die erfindungsgemäßen Verbindungen können wie nachstehend beschrieben hergestellt werden. Die nachfolgenden Beispiele dienen der näheren Erläuterung der Erfindung. Durch analoge Vorgehensweise unter Verwendung analoger Reagenzien zu den in den Beispielen enthaltenen Angaben lassen sich weitere Verbindungen der allgemeinen Formel I erhalten.

Diejenigen Seitenketten R¹¹, die keine Schwefelfunktion enthalten, lassen sich analog wie die entsprechenden 7α-ständigen Seitenketten der in der PCT/EP98/08470 beschriebenen Verbindungen aufbauen, wobei jetzt von dem hier in Beispiel 1d beschriebenen 11β-(5-Chlorpentyl)estra-1,3,5(10)-trien-3,17β-diol bzw. Beipiel 3a beschriebenen 11β-(5-Iodpentyl)estra-1,3,5(10)-trien-3,17β-diol auszugehen ist.

Eine Thiobrücke in der Seitenkette kann mit Natriumperiodat zum Sulfoxid oxidiert werden; mit einer Persäure als Oxidationsmittel. z.B. *m*-Chlorperbenzoesäure, werden aus den Sulfiden die Sulfone erhalten.

Die Verseifung von Estergruppierungen sowie Veresterung und Veretherung freier Hydroxygruppen erfolgt jeweils nach etablierten Verfahren der organischen Chemie. Durch Beachtung der unterschiedlichen Reaktivität der veresterten und freien 3- und 17-Hydroxygruppe lassen sich die 3,17-Diester selektiv in 3-Position spalten und die 3-Hydroxy-17-acyloxy-Verbindung läßt sich dann gezielt in 3-Position weiter funktionalisieren; genauso gut ist es möglich, die 3,17-Dihydroxyverbindung selektiv nur in 3-Position zu verestern oder zu verethern und dann gezielt in 17-Position einen anderen als bereits in 3-Stellung sich befindenden Rest einzuführen.

Die Säureadditionssalze der Verbindungen der allgemeinen Formel I lassen sich ebenfalls nach gängigen Verfahren aus den Verbindungen der allgemeinen Formel I herstellen.

Die nachfolgenden Beispiele dienen der näheren Erläuterung der Erfindung:

### Beispiel 1

### 11β-[5-(Methyl{3-[(4,4,5,5,5-pentafluorpentyl)sulfanyl]propyl}amino)pentyl]estra-1,3,5(10)-trien-3,17β-diol

### a) 11β-[5-(tert-Butyldimethylsilyloxy)pentyl]-3,3-(2,2-dimethyltrimethylendioxy)-5α-estr-9-en-5,17β-diol

1,82 g Magnesium in 15 ml absolutem Tetrahydrofuran werden unter Stickstoff mit einer Lösung von 21,1 g 1-Brom-5-tert.-butyldimethylsilyloxypentan [Tetrahedron Letters 1982, 4147-4150] in 40 ml absolutem Tetrahydrofuran zum Grignardreagenz umgesetzt. Bei 0 °C gibt man 0,25 g Kupfer-(I)-chlorid hinzu und rührt 15 Minuten nach, bevor man eine Lösung von 9,36 g 3,3-(2,2-Dimethyltrimethylendioxy)-5,10α-epoxy-5α-estr-9(11)-en-17β-ol [Neef G. et. al., Tetrahedron, (1993), 49, S. 833-840)] in 40 ml absolutem Tetrahydrofuran so zutropft, dass die Innentemperatur nicht über 8 °C ansteigt. Nach beendeter Zugabe wird für 90 Minuten bei Eisbadkühlung nach-gerührt. Zur Aufarbeitung wird das Reaktionsgemisch unter Rühren auf gesättigte Ammoniumchloridlösung gegeben, dreimal mit Essigester extrahiert, die vereinigten Essigesterphasen mit gesättigter Natriumchloridlösung gewaschen, über Natrium-sulfat getrocknet und im Vakuum zur Trockne eingeengt. Präparative Säulenchro-matographie an Kieselgel mit Hexan/Essigester als Eluenten liefert 5,02 g 11β-[5-(*tert*-Butyldimethylsilyloxy)pentyl]-3,3-(2,2-dimethyltrimethylendioxy)-5α-estr-9-en-5,17β-diol als Schaum.

### b) 17β-Hydroxy-11β-(5-hydroxypentyl)estra-4,9-dien-3-on

4,96 g 11β-[5-(*tert*-Butyldimethylsilyloxy)pentyl]-3.3-(2,2-dimethyltrimethylendioxy)-5α-estr-9-en-5,17β-diol in 36 ml Tetrahydrofuran werden mit 40 ml Eisessig und 20 ml Wasser 3 Stunden bei 50 °C Badtemperatur unter Stickstoff gerührt. Dann wird vorsichtig auf gesättigte Natriumhydrogencarbonatlösung gegossen, dreimal mit Essigester extrahiert, die vereinigten organischen Phasen mit gesättigter Natrium-chloridlösung gewaschen, über Natriumsulfat getrocknet und im Vakuum zur Trockne eingeengt. Durch präparative Säulenchromatographie an Kieselgel mit Hexan/Aceton als Eluenten erhält man 3,57 g 17β-Hydroxy-11β-(5-hydroxypentyl)estra-4,9-dien-3-on als Schaum.

### c) 11β-(5-Hydroxypentyl)-9ξ-estra-1,3,5(10)-trien-3,17β-diol

Eine Lösung von 1,08 g 17β-Hydroxy-11β-(5-hydroxypentyl)estra-4,9-dien-3-on in 19 ml Ethanol wird mit 0,19 g Palladium auf Aktivkohle 20 Stunden bei 100 °C Badtem-peratur unter Rückfluß erhitzt. Nach dem Abkühlen wird über Celite filtriert, mit Essigester nachgewaschen, das Filtrat zur Trockne eingeengt und an Kieselgel mit Hexan/Aceton chromatographiert. Umkristallisation aus Methylenchlorid führt zu 0,46 g 11β-(5-Hydroxypentyl)-9ξ-estra-1,3,5(10)-trien-3,17β-diol vom Schmelzpunkt 152 °C.

### d) 11β-(5-Chlorpentyl)estra-1,3,5(10)-trien-3,17β-diol

426 mg 11β-(5-Hydroxypentyl)-9ξ-estra-1,3,5(10)-trien-3,17β-diol werden in 7,2 ml Tetrachlorkohlenstoff und 2,4 ml Acetonitril suspendiert, mit 682 mg Triphenyl-phosphin versetzt und 90 Minuten bei Raumtemperatur gerührt. Zur Aufarbeitung wird die Reaktionslösung mit Methylenchlorid versetzt, mit gesättigter Natriumhydrogencarbonatlösung und gesättigter Natriumchloridlösung gewaschen, über Natriumsulfat getrocknet und im Vakuum eingeengt. Durch präparative Säulenchromatographie an Kieselgel mit Methylenchlorid/Essigester kann das 9β-Isomere abgetrennt werden. Anschließende Kristallisation aus Methylenchlorid liefert 238 mg 11 β-(5-Chlorpentyl)estra-1,3,5(10)-trien-3,17β-diol vom Schmelzpunkt 159 °C.

### e) 11β-[5-(Methyl{3-[(4,4,5,5,5-pentafluorpentyl)sulfanyl]propyl}amino)pentyl]estra-1,3,5(10)-trien-3,17β-diol

189 mg 11β-(5-Chlorpentyl)estra-1,3,5(10)-trien-3,17β-diol werden in 3 ml getrock-netem Dimethylformamid gelöst, mit 598 mg Methyl (3-[(4,4,5,5,5-pentafluorpentyl)-sulfanyl]propyl)amin [DE 196 35 525.7] versetzt und 24 Stunden bei 100 °C unter Stickstoff gerührt. Nachdem das Reaktionsgemisch abgekühlt ist, wird mit Essigester verdünnt, einmal mit gesättigter Natriumhydrogencarbonatlösung und zweimal mit gesättigter Natriumchloridlösung gewaschen, über Natriumsulfat getrocknet und im Vakuum eingeengt. Nach präparativer Säulenchromatographie an Kieselgel mit Essigester/Methanol als Eluenten erhält man 222 mg 11β-[5-(Methyl{3-[4,4,5,5,5-pentafluorpentyl)sulfanyl]propyl)amino)pentyl]estra-1,3,5(10)-trien-3,17β-diol als Schaum, [α]_{D}²² = + 72,1 ° (c = 0,172 in Chloroform).

### Beispiel 2

### 11β-(5-{3-[(4,4,5,5,5-Pentafluorpentyl)sulfanyl)propylamino}pentyl)estra-1,3,5(10)-trien-3,17β-diol

98 mg 11β-(5-Chlorpentyl)estra-1,3,5(10)-trien-3,17β-diol werden in 1,5 ml getrock-netem Dimethylformamid gelöst, mit 3 ml 3-[(4,4,5,5,5-Pentafluorpentyl)sulfanyl]-propylamin versetzt und 16 Stunden bei 80 °C Badtemperatur unter Stickstoff gerührt. Nach dem Abkühlen der Reaktionslösung wird mit Essigester verdünnt, einmal mit Wasser und zweimal mit gesättigter Natriumchloridlösung gewaschen, über Natriumsulfat getrocknet und im Vakuum eingeengt. Präparative Säulenchroma-tographie an Kieselgel mit Methylenchlorid/Methanol unter Zusatz von Ammoniak führt zu 31 mg 11β-(5-{3-[(4,4,5,5,5-Pentafluorpentyl)sulfanyl]propylamino}pentyl)-estra-1,3,5(10)-trien-3,17β-diol als Schaum.

### Beispiel 3

### 11β-[5-(Methyl(3-[(2-pyridylmethyl)sulfanyl]propyl}amino)pentyl]estra-1,3,5(10)-trien-3,17β-diol

### a) 11β-(5-Iodpentyl)estra-1,3,5(10)-trien-3,17β-diol

1,46 g 11β-(5-Chlorpentyl)estra-1,3,5(10)-trien-3,17β-diol werden in 24 ml Ethylmethylketon gelöst, mit 1,82 g Natriumiodid versetzt und über Nacht bei 90 °C Badtemperatur gerührt. Zur Aufarbeitung wird das Reaktionsgemisch auf Wasser gegeben, dreimal mit Essigester extrahiert, mit Natriumthiosulfatlösung gewaschen, über Magnesiumsulfat getrocknet und im Vakuum eingeengt. Man erhält 1,86 g 11β-(5-Iodpentyl)estra-1,3,5(10)-trien-3,17β-diol als Rohprodukt, welches ohne weitere Aufreinigung in der nächsten Reaktion eingesetzt wird.

### b) 11β-[5-(Methyl{3-[(2-pyridylmethyl)sulfanyl]propyl}amino)pentyl]estra-1,3,5(10)-trien-3,17β-diol

1,0 g 11β-(5-Iodpentyl)estra-1,3,5(10)-trien-3,17β-diol und 2,47 g Methyl{3-[(2-pyridylmethyl)sulfanyl]propyl}amin werden in 21 ml N-Methylpyrrolidon gelöst und 3 Stunden bei 80 °C gerührt. Nachdem die Reaktionslösung auf Raumtemperatur abgekühlt ist, wird der Ansatz auf halbgesättigte Natriumchloridlösung gegeben, dreimal mit Diethylether extrahiert, die vereinigten organischen Phasen mit Wasser und gesättigter Natriumchloridlösung gewaschen, über Magnesiumsulfat getrocknet und im Vakuum eingeengt. Präparative Säulenchromatographie an Kieselgel mit Methylenchlorid/Methanol als Eluenten liefert 0,69 g 11β-[5-(Methyl{3-[(2-pyridylmethyl)sulfanyl]propyl }amino)pentyl]estra-1,3,5(10)-trien-3,17β-diol als Schaum, [α]_{D}²²= + 62,2 ° (c = 0,519 in Chloroform).

### Beispiel 4

### 11β-[5-(Methyl {3-[(2-pyridylmethyl)sulfinyl]propyl}amino)pentyl]estra-1,3,5(10)-trien-3,17β-diol

250 mg 11β-[5-(Methyl{3-[(2-pyridylmethyl)sulfanyl]propyl}amino)pentyl]estra-1,3,5(10)-trien-3,17β-diol werden in 8,1 ml Methanol und 0,44 ml Wasser gelöst, mit 161 mg Natriummetaperiodat versetzt und bei Raumtemperatur 16 Stunden gerührt. Zur Aufarbeitung wird das Reaktionsgemisch in halbgesättigte Natriumchloridlösung gegeben, dreimal mit Methylenchlorid extrahiert, die vereinigten organischen Phasen über Magnesiumsulfat getrocknet und im Vakuum eingeengt. Präparative Säulen-chromatographie an Kieselgel mit Methylenchlorid/Methanol als Eluenten liefert 53 mg 11β-[5-(Methyl{3-[(2-pyridylmethyl)sulfinyl]propyl}amino)pentyl]estra-1,3,5(10)-trien-3,17β-diol als Schaum, [α]_{D}²²= + 58,9 °(c = 0,501 in Chloroform).

### Beispiel 5

### 11β-[5-(Methyl{3-[4-(trifluormethyl)benzylsulfanyl]propyl}amino)pentyl]estra-1,3,5(10)-trien-3,17β-diol

839 mg 11β-(5-Iodpentyl)estra-1,3,5(10)-trien-3,17β-diol und 1,42 g Methyl{3-[4-(trifluormethyl)benzylsulfanyl]propyl}amin werden in 18 ml N-Methylpyrrolidon gelöst und 90 Minuten bei 80 °C Badtemperatur gerührt. Nachdem die Reaktionslösung auf Raumtemperatur abgekühlt ist, wird der Ansatz auf halbgesättigte Natriumchlorid-lösung gegeben, dreimal mit Diethylether extrahiert, die vereinigten organischen Phasen mit Wasser und gesättigter Natriumchloridlösung gewaschen, über Magnesiumsulfat getrocknet und im Vakuum eingeengt. Präparative Säulenchroma-tographie an Kieselgel mit Methylenchlorid/Methanol als Eluenten liefert 0,94 g 11β-[5-(Methyl {3-[4-(trifluortnethyl)benzylsulfanyl]propyl)amino)pentyl]estra-1,3,5(10)-trien-3,17β-diol als Schaum, [α]_{D}²² =+ 75,0 ° (c = 0,5 in Chloroform).

### Beispiel 6

### 11β-[5-(Methyl{3-[4-(trifluormethyl)benzylsulfinyl]propyl}amino)pentyl]estra-1,3,5(10)-trien-3,17β-diol

630 mg 11β-[5-(Methyl{3-[4-(trifluormethyl)benzylsulfanyl]propyl}amino)pentyl]estra-1,3,5(10)-trien-3,17β-diol werden in 17,4 ml Methanol und 0,95 ml Wasser gelöst, mit 329 mg Natriummetaperiodat versetzt und bei Raumtemperatur 16 Stunden gerührt. Zur Aufarbeitung wird das Reaktionsgemisch in halbgesättigte Natriumchloridlösung gegeben, dreimal mit Methylenchlorid extrahiert, die vereinigten organischen Phasen über Magnesiumsulfat getrocknet und im Vakuum eingeengt. Präparative Säulen-chromatographie an Kieselgel mit Methylenchlorid/Methanol als Eluenten liefert 216 mg 11β-[5-(Methyl{3-[4-(trifluormethyl)benzylsulfinyl]propyl}amino)pentyl]estra-1,3,5(10)-trien-3,17β-diol als Schaum, [α]_{D}²² = + 54,1 ° (c = 0,501 in Chloroform).

### Beispiel 7

### 11β-{5-[(2S)-2-{[4-(Trifluormethyl)phenyl]sulfanylmethyl}pyrrolidin-1-yl]pentyl}estra-1,3,5(10)-trien-3,17β-diol

820 mg 11β-(5-Iodpentyl)estra-1,3,5(10)-trien-3,17β-diol und 458 mg (S)-2-{[4-(Trirluormethyl)phenyl]sulfanylmethyl}pyrrolidin werden in 16 ml N-Methylpyrrolidon gelöst und 3 Stunden bei 90 °C Badtemperatur gerührt. Nachdem die Reaktions-lösung auf Raumtemperatur abgekühlt ist, wird der Ansatz auf halbgesättigte Natriumchloridlösung gegeben, dreimal mit Essigester extrahiert, die vereinigten organischen Phasen dreimal mit Wasser gewaschen, über Magnesiumsulfat getrocknet und im Vakuum eingeengt. Präparative Säulenchromatographie an Kieselgel mit Methylenchlorid/Methanol als Eluenten liefert 561 mg 11β-{5-[(2S)-2-{[4-(Trifluormethyl)phenyl]sulfanylmethyl) pyrrolidin-1-yl]pentyl }estra-1,3,5(10)-trien-3,17β-diol als Schaum, [α]_{D}²² = + 33,1 °(c = 0,5195 in Chloroform).

### Beispiel 8

### 11β-{5-[(2S)-2-{[4-(Trifluormethyl)phenyl]sulfinylmethyl}pyrrolidin-1-yl]pentyl estra-1,3,5(10)-trien-3,17β-diol

975 mg 11β-(5-Iodpentyl)estra-1,3,5(10)-trien-3,17β-diol und 540 mg (S)-2-{[4-(Trifluormethyl)phenyl]sulfinylmethyl}pyrrolidin werden in 19 ml N-Methylpyrrolidon gelöst und 3 Stunden bei 90 °C Badtemperatur gerührt. Nachdem die Reaktions-lösung auf Raumtemperatur abgekühlt ist, wird der Ansatz auf halbgesättigte Natriumchloridlösung gegeben, dreimal mit Essigester extrahiert, die vereinigten organischen Phasen zweimal mit Wasser gewaschen, über Magnesiumsulfat getrocknet und im Vakuum eingeengt. Chromatographie an Kieselgel mit Methylen-chlorid/Methanol liefert 222 mg 11β-{5-[(2S)-2-{[4-(Trifluormethyl)phenyl]sulfinyl-methyl}pyrrolidin-1-yl]pentyl}estra-1,3.5(10)-trien-3,17β-diol als Schaum, [α]_{D}²²= + 42,6 ° (c = 0,5145 in Chloroform).

### Herstellung der Reagentien

### 3-[(4,4,5,5,5-Pentafluorpentyl)sulfanyl]propylamin

25 g S-(4,4,5,5,5-Pentafluorpentyl)thioacetat [Singh S. M. et. al., Tet. Lett., (1994), 35, S. 9141-9144] werden bei 0 °C in 250 ml absolutem Acetonitril vorgelegt und tropfenweise mit 39,2 ml einer 30 %igen Natriummethylatlösung versetzt. Es wird noch 15 Minuten in der Kälte nachgerührt, bevor 23,2 g 3-Brompropylamin Hydrobromid portionsweise eingetragen werden. Anschließend wird 1 Stunde bei 0 °C nachgerührt. Dann wird das Reaktionsgemisch auf Wasser gegeben, dreimal mit Diethylether extrahiert, neutral gewaschen, über Magnesiumsulfat getrocknet und im Vakuum eingeengt. Nach präparativer Säulenchromatographie an Kieselgel mit Methylenehlorid/Methanol unter Zusatz von Ammoniak als Eluenten, erhält man 22,54 g 3-[(4,4,5,5,5-Pentafluorpentyl)sulfanyl]propylamin.

### Methyl{3-[(2-pyridylmethyl)sulfanyl]propyl}amin

### a) S-(2-Pyridylmethyl)thioacetat

10,0 g 2-(Chlormethyl)pyridin Hydrochlorid werden in 100 ml Aceton vorgelegt, mit 14,0 g Kaliumthioacetat versetzt und bei 80 °C 2 Stunden unter Rückfluß gekocht. Zur Aufarbeitung wird das Reaktionsgemisch mit Essigester verdünnt und auf Wasser gegeben. Es wird dreimal mit Essigester extrahiert, die vereinigten org-anischen Phasen mit gesättigter Natriumhydrogencarbonatlösung und mit gesättigter Natriumchloridlösung gewaschen, über Magnesiumsulfat getrocknet und im Vakuum eingeengt. Nach präparativer Säulenchromatographie an Kieselgel mit Hexan/Essigester als Eluenten erhält man 9,78 g S-(2-Pyridylmethyl)thioacetat.

### b) 2-[(3-Chlorpropyl)sulfanylmethyl]pyridin

9,78 g S-(2-Pyridylmethyl)thioacetat in 90 ml absolutem Methanol werden unter Eisbadkühlung tropfenweise mit 10,9 ml einer 30 %igen methanolischen Natriummethylatlösung versetzt und nach beendeter Zugabe 20 Minuten nachgerührt. Dann wird bei 4 °C 8,6 ml 1-Brom-3-chlorpropan zugetropft und 2 Stunden in der Kälte und 1 Stunde bei Raumtemperatur nachgerührt. Anschließend wird das Reaktions-gemisch auf Wasser gegeben, dreimal mit Essigester extrahiert, die vereinigten organischen Phasen mit gesättigter Natriumchloridlösung gewaschen, über Magnesiumsulfat getrocknet und im Vakuum eingeengt. Präparative Säulenchroma-tographie an Kieselgel mit Hexan/Essigester als Eluenten liefert 11,7 g 2-[(3-Chlor-propyl)sulfanylmethyl]pyridin.

### c) Methyl{3-[(2-pyridylmethyl)sulfanyl]propyl}amin

In einem Druckgefäß werden 5,85 g 2-[(3-Chlorpropyl)sulfanylmethyl]pyridin in 33,5 ml Tetrahydrofuran mit 11,7 g Natriumiodid versetzt. Anschließend werden bei -20 °C 17,05 g Methylamin einkondensiert und über Nacht im Druckgefäß auf 50 °C Badtemperatur erwärmt. Nachdem das Reaktionsgefäß bei - 20 °C geöffnet wurde, läßt man auf Raumtemperatur kommen, um überschüssiges Methylamin abdampfen zu lassen. Die Reaktionslösung wird in verdünnte Natriumchloridlösung gegeben, dreimal mit Methylenchlorid extrahiert, die vereinigten organischen Phasen mit gesättigter Natriumchloridlösung gewaschen, über Magnesiumsulfat getrocknet und im Vakuum eingeengt. Man erhält 5,7 g Methyl {3-[(2-pyridylmethyl)sulfanyl]propyl}-amin als Rohprodukt.

### Methyl{3-[4-(trifluormethyl)benzylsulfanyl]propyl}amin

### a) 1-[(3-Brompropyl)sulfanylmethyl]-4-(trifluormethyl)benzol

25,0 g 4-(Trifluormethyl)benzylbromid werden in 84 ml Acetonitril vorgelegt, mit 8,3 ml Trimethylensulfid versetzt und 48 Stunden bei Raumtemperatur gerührt. Dann wird das Reaktionsgemisch zur Trockne eingeengt und an Kieselgel mit Hexan/ Methylenchlorid chromatographiert. Man erhält 29,47 1-[(3-Brompropyl)sulfanyl-methyl]-4-(trifluormethyl)benzol als Öl.

### b) Methyl{3-[4-(trifluormethyl)benzylsulfanyl]propyl}amin

Zu einer Lösung von 10,0 g 1-[(3-Brompropyl)sulfanylmethyl]-4-(trifluormethyl)benzol in 37,0 ml Tetrahydrofuran werden bei -20 °C 18,8 g Methylamin kondensiert und über Nacht im Druckgefäß bei Raumtemperatur gerührt. Nachdem das Reaktions-gefäß bei - 20 °C geöffnet wurde, läßt man auf Raumtemperatur kommen, um überschüssiges Methylamin abdampfen zu lassen. Die Reaktionslösung wird in verdünnte Natriumchloridlösung gegeben, dreimal mit Methylenchlorid extrahiert, die vereinigten organischen Phasen mit gesättigter Natriumchloridlösung gewaschen, über Magnesiumsulfat getrocknet und im Vakuum eingeengt. Man erhält 8,32 g Methyl {3-[4-(trifluormethyl)benzylsulfanyl]propyl}amin als Rohprodukt.

### 1-Brom-4-[2-(tert-butyldimethylsilyloxy)ethyl]benzol

70 g 2-(4-Bromphenyl)ethanol werden in 350 ml absolutem Tetrahydrofuran gelöst und mit 4,97 g Imidazol versetzt. Anschließend werden 55 g *tert*-Butyldimethylsilyl-chlorid in 230 ml absolutem Tetrahydrofuran zugetropft und 90 Minuten bei Raum-temperatur gerührt. Zur Aufarbeitung wird das Reaktionsgemisch in Eiswasser gegeben, dreimal mit Diethylether extrahiert, mit Wasser gewaschen, über Magnesiumsulfat getrocknet und im Vakuum eingeengt. Präparative Säulenchro-matographie an Kieselgel mit Hexan/Essigester als Eluenten liefert 111g 1-Brom-4-[2-(*tert*-butyldimethylsilyloxy)ethyl]benzol als klares Öl.

### (S)-2-{[4-(Trifluormethyl)phenyl]sulfanylmethyl}pyrrolidin

### a) (S)-tert-Butyl-2-{[4-(trifluormethyl)phenyl]sulfanylmethyl}pyrrolidin-1-carboxylat

Zu einer Lösung aus 1,4 g 4-(Trifluormethyl)thiophenol in 15 ml absolutem Aceton werden unter Inertgas bei 0 °C 1,93 ml einer 30 %igen Natriummethylatlösung langsam zugetropft. Nach beendeter Zugabe wird noch 30 Minuten in der Kälte nachgerührt, bevor 1,85 g (S)-*tert-*Butyl-2-(brommethyl)pyrrolidin-1-carboxylat [Katzenellenbogen J. A. et. al.; J. Med. Chem. (1994), 37, S. 928-937] in 5 ml absolutem Aceton zugetropft werden. Anschließend wird 30 Minuten in der Kälte und 12 Stunden bei Raumtemperatur nachgerührt. Zur Aufarbeitung wird das Reaktions-gemisch auf Wasser gegeben, dreimal mit Diethylether extrahiert, zweimal mit gesättigter Natriumchloridlösung gewaschen, über Magnesiumsulfat getrocknet und im Vakuum eingeengt. Präparative Säulenchromatographie an Kieselgel mit Hexan/Essigester als Eluenten liefert 1,8 g (S)-*tert*-Butyl-2-{[4-(trifluormethyl)phenyl]sulfanylmethyl}pyrrolidin-1-carboxylat als Öl.

### b) (S)-2-{[4-(Trifluormethyl)phenyl]sulfanylmethyl}pyrrolidin

920 mg (S)-*tert*-Butyl-2-{[4-(trifluormethyl)phenyl]sulfanylmethyl}pyrrolidin-1-carboxylat, werden zu einer auf 0 °C gekühlten Mischung aus 7,2 ml Trifluoressig-säure, 0,22 ml Triisopropylsilan und 0,30 ml Wasser gegeben und für 1 Stunde bei 0 °C gerührt. Anschließend wird die Reaktion mit 10%iger Kaliumhydroxidlösung auf pH 10 eingestellt, dreimal mit Methylenchlorid extrahiert, mit gesättigter Natrium-chloridlösung gewaschen, über Magnesiumsulfat getrocknet und im Vakuum eingeengt. Man erhält 458 mg (S)-2-{[4-(Trifluormethyl)phenyl]sulfanylmethyl }-pyrrolidin als Rohprodukt.

### (S)-2-{[4-(Trifluormethyl)phenyl]sulfinylmethyl}pyrrolidin

### a) (S)-tert-Butyl-2-{[4-(trifluormethyl)phenyl]sulfinylmethyl}pyrrolidin-1-carboxylat

1,0 g (S)-*tert*-Butyl-2-{[4-(trifluormethyl)phenyl]sulfanylmethyl}pyrrolidin-1-carboxylat wird in 48 ml Methanol und 2,6 ml Wasser gelöst, mit 833 mg Natriummetaperiodat versetzt, 2 Tage bei Raumtemperatur und 1 Stunde bei 50 °C gerührt. Zur Aufar-beitung wird das Reaktionsgemisch in halbgesättigte Natriumchloridlösung gegeben, dreimal mit Methylenchlorid extrahiert, mit gesättigter Natriumchloridlösung gewaschen, über Magnesiumsulfat getrocknet und im Vakuum eingeengt. Der Rückstand wird durch präparative Säulenchromatographie mit Hexan/Aceton als Eluenten gereinigt. Man erhält 929 mg (S)-*tert*-Butyl-2-{[4-(trifluormethyl)phenyl]-sulfinylmethyl)pyrrolidin-1-carboxylat.

### b) (S)-2-([4-(Trifluormethyl)phenyl]sulfinylmethyl)pyrrolidin

920 mg (S)-*tert*-Butyl-2-{[4-(trifluormethyl)phenyl]sulfinylmethyl}pyrrolidin-1-carboxylat, werden zu einer auf 0 °C gekühlten Mischung aus 7,2 ml Trifluoressig-säure, 0,22 ml Triisopropylsilan und 0,30 ml Wasser gegeben, kurzzeitig auf Raumtemperatur erwärmt, bis das Edukt gelöst ist und dann für 30 Minuten bei 0 °C gerührt. Anschließend wird die Reaktion mit 10%iger Kaliumhydroxidlösung auf pH 10 eingestellt, dreimal mit Methylenchlorid extrahiert, mit gesättigter Natriumchlorid-lösung gewaschen, über Magnesiumsulfat getrocknet und im Vakuum eingeengt. Man erhält 549 mg (S)-2-{[4-(Trifluormethyl)phenyl]sulfinyl}pyrrolidin als Rohprodukt.

## Patentansprüche

1. 11β-langkettig-substituierte Estratriene der allgemeinen Formel I 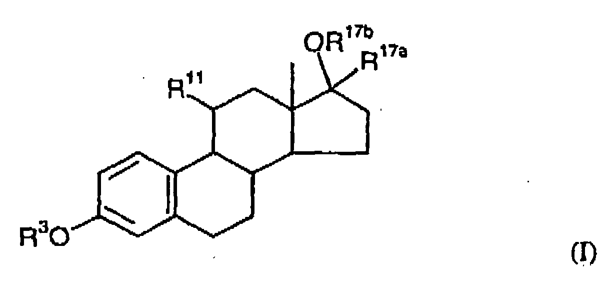 worin
R³ ein Wasserstoffatom, einen Kohlenwasserstoffrest mit bis zu 8 Kohlenstoffatomen oder einen Rest der Teilformel R^{3'}-C(O)-, worin R^{3'} ein Wasserstoffatom oder ein Kohlenwasserstoffrest mit bis zu 8 Kohlenstoffatomen oder ein Phenylrest ist, bedeutet,
R¹¹ einen Rest der Formel -A-B-Z-R²⁰ bedeutet,
worin
A für eine direkte Bindung und
B für eine gerad- oder verzweigtkettige Alkylen-, Alkenylen- oder Alkinylengruppe mit 4, 5 oder 6 Kohlenstoffatomen stehen,
Z für -NR²¹- und R²¹ für eine C₁-C₃-Alkylgruppe stehen,
wobei R²⁰
ein Wasserstoffatom,
eine gerad- oder verzweigtkettige Alkyl-, Alkenyl- oder Alkinylgruppe mit bis zu 10 Kohlenstoffatomen,
wobei R²⁰ und R²¹ aber beide nicht gleichzeitig Methyl, sein dürfen,
oder eine der Gruppierungen
-D-CₙF₂ₙ₊₁, wobei D eine gerad- oder verzweigtkettige Alkylen-, Alkenylen- oder Alkinylengruppe mit bis zu 8 Kohlenstoffatomen und n eine ganze Zahl von 1 bis 8 ist,
D-Aryl, wobei D die bereits angegebene Bedeutung hat und Aryl für einen gegebenenfalls ein- oder zweifach substituierten Phenyl-, 1- oder 2-Naphthyl- oder einen Heteroarylrest steht,
-L-CH=CF-CₚF₂ₚ₊₁, wobei L eine gerad- oder verzweigtkettige Alkylen-, Alkenylen- oder Alkinylengruppe mit bis zu 7 Kohlenstoffatomen und p eine ganze Zahl von 1 bis 7 ist,
wobei in den drei vorstehenden Fällen in D bzw. L eine Methylengruppe durch ein Schwefelatom, eine Sulfon- oder Sulfoxidgruppe ersetzt sein kann,
-D-O-(CH₂)_{q}-Aryl, wobei D und Aryl die bereits angegebenen Bedeutungen haben und q 0, 1, 2 oder 3 ist,
-D-O-(CH₂)ᵣ-CₙF₂ₙ₊₁, wobei D und n die bereits angegebenen Bedeutungen haben und r für eine ganze Zahl von 1 bis 5 steht,
bedeutet.
wobei außerdem in allen betreffenden vorstehenden Fällen R²¹ gemeinsam mit D unter Einschluß des Stickstoffatoms einen dann in 2- oder 3-Stellung substituierten Pyrrolidinring bilden kann,
oder
R²⁰ und R²¹ mit dem Stickstoffatom, an das sie gebunden sind, einen gesättigten oder ungesättigten Heterocyclus mit 5 oder 6 Kettengliedern, der gegebenenfalls ein oder zwei weitere Heteroatome, ausgewählt aus Stickstoff, Sauerstoff und Schwefel, enthält und gegebenenfalls substituiert ist, bilden,
R^{17a} in α- oder β-Position ein Wasserstoffatom, eine C₁₋₅-Alkyl-, eine C₂₋₅-Alkenyl- oder eine C₂₋₅-Alkinylgruppe oder eine Trifluormethylgruppe oder gemeinsam mit dem Rest OR^{17b} ein Ketosauerstoffatom bedeutet, und
R^{17b} ein Wasserstoffatom oder einen Rest der Teilformel R^{17'}-C(O)-, worin R^{17'} ein Wasserstoffatom oder ein Kohlenwasserstoffrest mit bis zu 8 Kohlenstoffatomen ist, bedeutet.

2. 11β-substituierte-Estratriene nach Anspruch 1, worin R³ ein Wasserstoffatom ist.

3. 11β-substituierte-Estratriene nach Anspruch 1, worin R³ ein Benzoylrest ist.

4. 11β-substituierte-Estratriene nach Anspruch 1, worin R^{17b} ein Wasserstoffatom ist

5. 11β-substituierte-Estratriene nach Anspruch 1, worin R¹¹ aus der Gruppe der folgenden Seitenketten ausgewählt ist
-(CH₂)₅N(CH₃)-(CH₂)3-S-(CH₂)₃C₂F₅
-(CH₂)₅NH-(CH₂)₃-S-(CH₂)₃C₂F₅
-(CH₂)₅N(CH₃)-(CH2)₃-S-CH₂-2-Pyridyl
-(CH₂)₅N(CH₃)-(CH₂)₃-SO-CH₂-2-Pyridyl
-(CH₂)₃N(CH₃)-(CH₂)₃-S-CH₂-p-CF₃-Phenyl
-(CH₂)₅N(CH₃)-(CH₂)₃-SO-CH₂-p-CF₃-Phenyl
-(CH₂)₅-[2-Pyrrolidin-1-yl]-CH₂-S-p-CF₃-Phenyl
-(CH₂)₅-[2-Pyrrolidin-1-yl]-CH₂-SO-CF₃-Phenyl
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₅F₁₇
-(CH₂)₅N(CH₃)H
-(CH₂)₃N(CH₃)(CH₂)₉H
-(CH₂)₅N(CH₃)CH₂CH=CF-C₂F₅
-(CH₂)₅N(CH₃)CH₂CH=CF-C₃F₇
-(CH₂)₅N(CH₃)CH₂CH=CF-C₅F₁₁
-(CH₂)₅N(CH₃)CH₂CH=CF-C₇F₁₅
-(CH₂)₅-1-Pyrrolidinyl
-(CH₂)₅N(CH₃)(CH₂)₃0Phenyl
-(CH₂)₅N(CH₃)(CH₂)₃OBenzyl
-(CH₃)₅N(CH₃)(CH₂)₃O(CH₂)₃C₂F₅
-(CH₂)₅N(CH₃)(CH₂)₃CH(CH₃)₂
-(CH₂)₅N(CH₃)(CH₂)₃-Pyridyl
-(CH₂)₅N(CH₃)(CH₂)₃-Phenyl
-(CH₂)₅N(CH₃)(CH₂)₂-p-Totyl
-(CH₂)₅N(CH₃)(CH₂)₂-p-Ethoxyphenyl
-(CH₂)₅N(CH₃)(CH₂)₃-p-Tolyl
-(CH₂)₅N(CH₃)(CH₂)₃-p-Chlorphenyl
-(CH₂)₅N(CH₃)(CH₂)₃-O-CH₂-Phenyl
-(CH₂)₅N(CH₃)(CH₂)₂-O-p-Br-Phenyl
-(CH₂)₅N(CH₃)(CH₂)₂-O-p-CF₃-Phenyl

6. 11β-langkettig substituierte Estratriene der allgemeinen Formel I, nämlich
11β-[5-(Methyl{3-[(4,4,5,5,5-pentafluorpentyl)sulfanyl]propyl}amino)pentyl]estra-1,3,5(10)-trien-3,17β-diol
11β-(5-[3-[(4,4,5,5,5-Pentafluorpentyl)sulfanyl]propylamino]pentyl)estra-1,3,5(10)-trien-3,17β-diol
11β-[5-(Methyl[3-[(2-pyridylmethyl)sulfanyl]propyl]amino)pentyl]estra-1,3,5(10)-trien-3,17β-diol
11β-[5-(Methyl{3-[(2-pyridylmethyl)sulfinyl]propyl}amino)pentyl]estra-1,3,5(10)-trien-3,17β-diol
11β-[5-(Methyl{3-[4-(trifluormethyl)benzylsulfanyl]propyl}amino)pentyl]estra-1,3,5(10)-bien-3,17β-diol
11β-[5-(Methyl{3-[4-(trifluormethyl)benzylsulfinyl]propyl}amino)pentyl]estra-1,3,5(10)-trien-3,17β-diol
11β-[5-[(2S)-2-{[4-(Trifluormethyl)phenyl]sulfanylmethyl}pyrrolidin-1-yl]pentyl]estra-1,3,5(10)-trien-3,17β-diol
11β-[5-[(2S)-2-(([4-(Trifluormethyl)phenyl]sulfinylmethyl)pyrrolidin-1-yl)pentyl]estra-1,3,5(10)-trien-3,17β-diol
11β-{5-[methyl-(8,8,9,9,9-pentafluor-nonyl)-amino]-pentyl}-estra-1,3,5(10)-trien-3,17β-diol
11β-[5-[methyl-nonyl-amino]-pentyl]-estra-1,3,5(10)-trien-3,17β-diol
11β-[5-[methyl-(9,9,10,10,10-pentafluor-decyl)-amino]-pentyl]-estra-1,3,5(10)-trien-3,17β-diol
11β-[6-[methyl-(8,8,9,9,9-pentafluor-nonyl)-amino]-hexyl}-estra-1,3,5(10)-trien-3,17β-diol
11β-[6-[methyl-(9,9,10,10,10-pentafluor-decyl)-amino]-hexyl]-estra-1,3,5(10)-trien3,17β-diol
11β-(5-(methyl-amino)-pentyl)-estra-1,3,5(10)-trien-3,17β-diol
11β-(5-pyrrolidin-1-yl-pentyl)-estra-1,3,5(10)-trien-3,17β-diol
11β{5-[methyl-(4,4,5,5,5-pentafluor-pentyl)-amino]-pentyl})-estra-1,3,5(10)-trien-3,17β-diol
11β-[5-[methyl-(4,4,5,5,6,6,7,7,8,8,9,9,9-tridecafluor-nonyl)-amino]-pentyl)-estra-1,3,5(10)-trien-3,17β-diol
11β-(5-[(4,4,5,5,6,6,7,7,8,8,9,9,10,10,11,11,11-heptadecafluor-undecyl)-methyl-amino]-pentyl)-estra-1,3,5(10)-trien-3,17β-diol
11β-(5-[methyl-(3,3,4,4,5,5,6,6,6-nonafluor-hexyl)-amino]-pentyl)-estra-1,3,5(10)-trien-3,17 β-diol
11β-{5-[methyl-(7,7,8,8,8-pentafluor-octyl)-amino]-pentyl}-estra-1,3,5(10)-trien-3,17β-diol
11β-{6-[methyl-(7,7,8,8,8-pentafluor-octyl)-amino]-hexyl}-estra-1,3,5(10)-trien-3,17β-diol
11β-{5-[methyl-(7,7,8,8,9,9,10,10,10-nonafluor-decyl)-amino]-pentyl}-estra-1,3,5(10)-trien-3,17β-diol
11β-{5-(methyl-(7,7,8,8,9,9,10,10,11,11,12,12,12-tridecafluor-dodecyl)-amino]-pentyl}-estra-1,3,5(10)-trien-3,17β-diol
11β-{5-[(7,7,8,8,9,9,10,10,11,11,12,12,13,13,14,14,14-heptadecafluor-tetradecyl)-methylamino]-pentyl}-estra-1,3,5(10)-trien-3,17β-diol
11β-{5-[(3,4,4,5,5,5-hexafluor-pent-2-enyl)-methyl-amino]-pentyl}-estra-1,3,5(10)-trien-3,17 β-diol
11β-{5-[(3,4,4,5,5,6,6,7,7,8,8,8-Dodecafluor-oct-2-enyl)-methyl-amino]-pentyl}-estra-1,3,5(10)-trien-3,17β-diol
11β{5-[(3,4,4,5,5,6,6,7,7,8,8,9,9,10,10,10-hexadecafluor-dec-2-enyl)-methyl-amino]-pentyl}-estra-1,3,5(10)-trien-3,17β-diol
11β-{5-[methyl-(3-phenoxy-propyl)-amino]-pentyl}-estra-1,3,5(10)-trien-3,17β-diol
11β-{5-[(3-Benzyloxy-propyl)-methyl-amino]-pentyl}-estra-1,3,5(10)-trien-3,17β-diol
11β-{5-[N-methyl-N-3-(4,4,5,5,5-pentafluorpentyloxy)-propylamino]-pentyl}-estra-1,3,5(10)-trien-3,17β-diol
11β-{5-[methyl-(2-p-tolyl-ethyl)-amino]-pentyl}-estra-1,3,5(10)-trien-3,17β-diol
11β-(5-{[2-(4-Ethoxy-phenyl)-ethyl]-methyl-amino}-pentyl)-estra-1,3,5(10)-trien-3,17β-diol
11β-{5-[methyl-(3-phenyl-propyl)-amino]-pentyl}-estra-1,3,5(10)-trien-3,17β-diol
11β-{5-[methyl-(3-pyridin-3-yl-propyl)-amino]-pentyl}-estra-1,3,5(10)-trien-3,17β-diol
11β-{5-[methyl-(3-p-tolyl-propyl)-amino]-pentyl}-estra-1,3,5(10)-trien-3,17β-diol
11β-(5-{[3-(4-Chlor-phenyl)-propyl]-methyl-amino}-pentyl)-estra-1,3,5(10)-trien-3,17β-diol
11β-(5-{[3-(4-Ethoxy-phenyl)-propyl]-methyl-amino}-pentyl)-estra-1,3,5(10)-trien-3,17β-diol
11β-{5-[methyl-(4-methyl-pentyl)-amino]-pentyl}-estra-1,3,5(10)-trien-3,17β-diol

7. Verwendung der Verbindungen der allgemeinen Formel I gemäß Anspruch 1 zur Herstellung von Arzneimitteln.

8. Pharmazeutische Präparate enthaltend mindestens eine Verbindung der allgemeinen Formel I gemäß Anspruch 1 sowie einen pharmazeutisch verträglichen Träger.

## Claims

1. 11β-long-chain-substituted estratrienes of general formula I 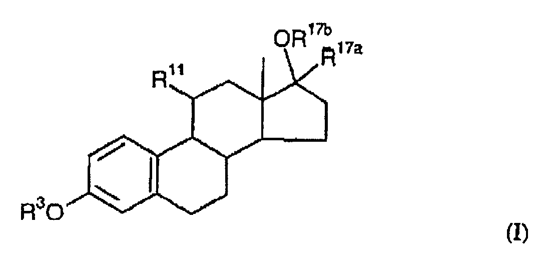 in which R³ means a hydrogen atom, a hydrocarbon radical with up to 8 carbon atoms or a radical of partial formula R^{3'}-C(O)-, in which R^{3'} means a hydrogen atom or a hydrocarbon radical with up to 8 carbon atoms or a phenyl radical, R¹¹ means a radical of formula -A-B-Z-R²⁰, in which A stands for a direct bond, and B stands for a straight-chain or branched-chain alkylene, alkenylene or alkinylene group with 4, 5 or 6 carbon atoms, Z stands for --NR²¹- and R²¹ stands for a C₁-C₃ alkyl group, wherein R²⁰ means a hydrogen atom, a straight-chain or branched-chain alkyl, alkenyl or alkinyl group with up to 10 carbon atoms, whereby R²⁰ and R²¹ may not both simultaneously mean methyl, however,
or one of the groupings
--D--CₙF₂ₙ₊₁, wherein D is a straight-chain or branched-chain alkylene, alkenylene or alkinylene group with up to 8 carbon atoms and n is an integer from 1 to 8, D-aryl, wherein D has the already indicated meaning, and aryl stands for a phenyl, 1- or 2-naphthyl radical or a heteroaryl radical that is optionally substituted in one or two places, --L--CH=CF--CₚF₂ₚ₊₁, wherein L is a straight-chain or branched-chain alkylene, alkenylene or alkinylene group with up to 7 carbon atoms and p is an integer from 1 to 7, wherein in the three cases above in D or L, a methylene group can be replaced by a sulfur atom, a sulfone group or a sulfoxide group, --D--O-(CH₂)_{q}-aryl, wherein D and aryl have the already indicated meanings, and q is 0, 1, 2 or 3, --D--O-(CH₂)ᵣ --CₙF₂ₙ₊₁, wherein D and n have the already indicated meanings, and r stands for an integer from 1 to 5, wherein in addition in all relevant cases above, R²¹ together with D with the inclusion of the nitrogen atom can then form a pyrrolidine ring that is substituted in 2- or 3-position, or R²⁰ and R²¹ with the nitrogen atom to which they are bonded form a saturated or unsaturated heterocyclic compound with 5 or 6 chain links, which optionally contains one or two additional heteroatoms, selected from nitrogen, oxygen and sulfur, and optionally is substituted, and R^{17a} in α- or β-position means a hydrogen atom, a C₁₋₅ alkyl, a C₂₋₅ alkenyl or a C₂₋₅ alkinyl group or a trifluoromethyl group, or together with the radical OR^{17b} means a keto-oxygen atom, and R^{17b} means a hydrogen atom or a radical of partial formula R^{17'}--C(O)--, in which R^{17'} means a hydrogen atom or a hydrocarbon radical with up to 8 carbon atoms.

2. 11β-substituted estratrienes according to Claim 1, in which R³ is a hydrogen atom.

3. 11β-substituted estratrienes according to Claim 1, in which R³ is a benzoyl radical.

4. 11β-substituted estratrienes according to Claim 1, in which R^{17b} is a hydrogen atom.

5. 11β-substituted estratrienes according to Claim 1, in which R¹¹ is selected from the group of the following side chains
-(CH₂)₅N(CH₃)-(CH₂)₃-S-(CH₂)₃C₂F₅
-(CH₂)₅NH-(CH₂)₃-S-(CH₂)₃C₂F₅
-(CH₂)₅N(CH₃)-(CH₂)₃-S-CH₂-2-Pyridyl
-(CH₂)₅N(CH₃)-(CH₂)₃-SO-CH₂-2-Pyridyl
-(CH₂)₅N(CH₃)-(CH₂)₃-S-CH₂-p-CF₃-Phenyl
-(CH₂)₅N(CH₃)-(CH₂)₃-SO-CH₂-p-CF₃-Phenyl
-(CH₂)₅-[2-Pyrrolidin-1-yl]-CH₂-S-p-CF₃-Phenyl
-(CH₂)₅-[2-Pyrrolidin-1-yl]-CH₂-SO-p-CF₃-Phenyl
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂) ₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅N(CH₃)CH₂CH=CF-C₂F₅
-(CH₂)₅N(CH₃)CH₂CH=CF-C₃F₇
-(CH₂)₅N(CH₃)CH₂CH=CF-C₅F₁₁
-(CH₂)₅N(CH₃)CH₂CH=CF-C₇F₁₅
-(CH₂)₅-1-Pyrrolidinyl
-(CH₂)₅N(CH₃)(CH₂)₃OPhenyl
-(CH₂)₅N(CH₃)(CH₂)₃OBenzyl
-(CH₂)₅N(CH₃)(CH₂)₃O(CH₂)₃C₂F₅
-(CH₂)₅N(CH₃)(CH₂)₃CH(CH₃)₂
-(CH₂)₅N(CH₃)(CH₂)₃-Pyridyl
-(CH₂)₅N(CH₃)(CH₂)₃-Phenyl
-(CH₂)₅N(CH₃)(CH₂)₂-p-Tolyl
-(CH₂)₅N(CH₃)(CH₂)₂-p-Ethoxyphenyl
-(CH₂)₅N(CH₃)(CH₂)₃-p-Tolyl
-(CH₂)₅N(CH₃)(CH₂)₃-p-Chlorophenyl
-(CH₂)₅N(CH₃)(CH₂)₃-O-CH₂-Phenyl
-(CH₂)₅N(CH₃)(CH₂)₂-O-p-Br-Phenyl
-(CH₂)₅(CH₃)(CH₂)₂-O-p-CF₃-Phenyl

6. 11β-long-chain-substituted estratrienes of the general formula I, namely
11β-[5-(methyl{3-[(4,4,5,5,5-pentafluoropentyl)sulfanyl]-propyl}amino)pentyl]estra-1,3,5(10)-triene-3,17β-diol
11β-(5-{3-[(4,4,5,5,5-pentafluoropentyl)sulfanyl]propyl-amino}pentyl)estra-1,3,5(10)-triene-3,17β-diol
11β-[5-(methyl{3-[(2-pyridylmethyl)sulfanyl]propyl}amino)pentyl]estra-1,3,5(10)-triene-3,17p-diol
11β-[5-(methyl{3-[(2-pyridylmethyl)sulfinyl]propyl}amino)pentyl]estra-1,3,5(10)-triene-3,17β-diol
11β-[5-(methyl{3-[4-(trifluoromethyl)benzylsulfanyl]-propyl}amino)pentyl]estra-1,3,5(10)-triene-3,17β-diol
11β-[5-(methyl{3-[4-(trifluoromethyl)benzylsulfinyl]-propyl}amino)pentyl]estra-1,3,5(10)-triene-3,17β-diol
11β-{5-[(2S)-2-{[4-(trifluoromethyl)phenyl]sulfanylmethyl}pyrrolidin-1-yl]pentyl}estra-1,3,5(10)-triene-3,17β-diol
11β-{5-[(2S)-2-{[4-(trifluoromethyl)phenyl]sulfinylmethyl}pyrrolidin-1-yl]pentyl}estra-1,3,5(10)-triene-3,17β-diol
11β-{5-[methyl-(8,8,9,9,9-pentafluoro-nonyl)-amino]-pentyl)-estra-1,3,5(10)-triene-3,17β-diol
11β-{5[methyl-nonyl-amino]-pentyl}-estra-1,3,5(10)-triene-3,17β-diol
11β-{5-[methyl-(9,9,10,10,10-pentafluoro-decyl)-amino]-pentyl}-estra-1,3,5(10)-triene-3,17β-diol
11β-{6-[methyl-(8,8,9,9,9-pentafluoro-nonyl)amino]-hexyl}-estra-1,3,5(10)-triene-3,17β-diol
11β-{6-[methyl-(9,9,10,10,10-pentafluoro-decyl)-amino]-hexyl}-estra-1,3,5(10)-triene-3,17β-diol
11β-(5-(methyl-amino)-pentyl)-estra-1,3,5(10)-triene-3,17β-diol
11β-(5-pyrrolidin-1-yl-pentyl)-estra-1,3,5(10)-triene-3,17β-diol
11β-{5-[methyl-(4,4,5,5,5-pentafluoro-pentyl)-amino]-pentyl}-estra-1,3,5(10)-triene-3,17β-diol
11β-{5-[methyl-(4,4,5,5,6,6,7,7,8,8,9,9,9-tridecafluoro-nonyl)-amino]-pentyl}-estra-1,3,5(10)-triene-3,17β-diol
11β-{5-[(4,4,5,5,6,6,7,7,8,8,9,9,10,10,11,11,11-heptadecafluoro-undecyl)-methyl-amino]-pentyl}-estra-1,3,5(10)-triene-3,17β-diol
11β-{5-[methyl-(3,3,4,4,5,5,6,6,6-nonafluoro-hexyl)-amino]-pentyl}-estra-1,3,5(10)-triene-3,17β-diol
11β-{5-[methyl-(7,7,8,8,8-pentafluoro-octyl)-amino]-pentyl}-estra-1,3,5(10)-triene-3,17β-diol
11β-{6-[methyl-(7,7,8,8,8-pentafluoro-octyl)-amino]-hexyl}-estra-1,3,5(10)-triene-3,17β-diol
11β-{5-[methyl-(7,7,8,8,9,9,10,10,10-nonafluoro-decyl)-amino]-pentyl}-estra-1,3,5(10)-triene-3,17β-diol
11β-{5-[methyl-(7,7,8,8,9,9,10,10,11,11,12,12,12-tridecafluoro-dodecyl)-amino]-pentyl}-estra-1,3,5(10)-triene-3,17β-diol
11β-{5-[(7,7,8,8,9,9,10,10,11,11,12,12,13,13,14,14,14-heptadecafluoro-tetradecyl)-methyl-amino]-pentyl}estra-1,3,5(10)-triene-3,17β-diol
11β-{5-[(3,4,4,5,5,5-hexafluoro-pent-2-enyl)-methylamino]-pentyl}-estra-1,3,5(10)-triene-3,17β-diol
11β-{5-[(3,4,4,5,5,6,6,7,7,8,8,8-dodecafluoro-oct-2-enyl)-methyl-amino]-pentyl}-estra-1,3,5(10)-triene-3,17β-diol
11β-{5-[(3,4,4,5,5,6,6,7,7,8,8,9,9,10,10,10-hexadecafluoro-dec-2-enyl)-methyl-amino]-pentyl}-estra-1,3,5(10)-triene-3,17β-diol
11β-{5-[methyl-(3-phenoxy-propyl)-amino]-pentyl}-estra-1,3,5(10)-triene-3,17β-diol
11β-{5-[(3-benzyloxy-propyl)-methyl-amino]-pentyl}-estra-1,3,5(10)-triene-3,17β-diol
11β-{5-[N-methyl-N-3-(4,4,5,5,5-pentafluoropentyloxy)-propylamino]-pentyl}-estra-1,3,5(10)-triene-3,17β-diol
11β-{5-[methyl-(2-p-tolyl-ethyl)-amino]-pentyl}-estra-1,3,5(10)-triene-3,17β-diol
11β-(5-{[2-(4-ethoxy-phenyl)-ethyl]-methyl-amino}-pentyl)-estra-1,3,5(10)-triene-3,17β-diol
11β-{5-[methyl-(3-phenyl-propyl)-amino]-pentyl}-estra-1,3,5(10)-triene-3,17β-diol
11β-{5-[methyl-(3-pyridin-3-yl-propyl)-amino]-pentyl}-estra-1,3,5(10)-triene-3,17β-diol
11β-{5-[methyl-(3-p-tolyl-propyl)-amino]-pentyl}-estra-1,3,5(10)-triene-3,17β-diol
11β-(5-{[3-(4-chloro-phenyl)-propyl]-methyl-amino}-pentyl)-estra-1,3,5(10)-triene-3,17β-diol
11β-(5-{[3-(4-ethoxy-phenyl)-propyl]-methyl-amino}-pentyl)-estra-1,3,5(10)-triene-3,17β-diol
11β-{5-[methyl-(4-methyl-pentyl)-amino]-pentyl}-estra-1,3,5(10)-triene-3,17β-diol

7. Use of compounds of the general formula I according to Claim 1 for the production of pharmaceutical agents.

8. Pharmaceutical preparation comprising at least one compound of general formula I according to Claim 1 and a pharmaceutically compatible vehicle.

## Revendications

1. Estratriènes substitués en 11β par de longues chaînes, de formule générale I 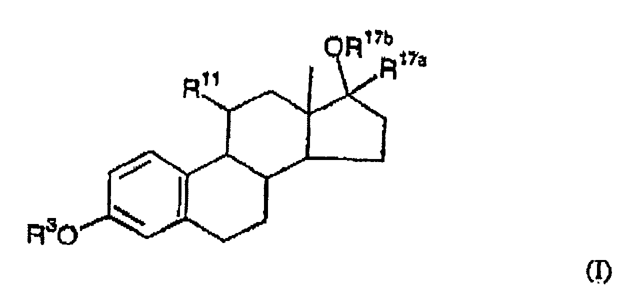 dans laquelle
R³ représente un atome d'hydrogène, un radical hydrocarboné ayant jusqu'à 8 atomes de carbone ou un radical de formule partielle R^{3'}-C(O)-, dans laquelle R³ représente un atome d'hydrogène ou un radical hydrocarboné ayant jusqu'à 8 atomes de carbone ou un radical phényle,
R¹¹ représente un radical de formule -A-B-Z-R²⁰,
dans laquelle
A représente une liaison directe et
B représente un groupe alkylène, alcénylène ou alcynylène à chaîne droite ou ramifiée ayant 4, 5 ou 6 atomes de carbone,
Z représente -NR²¹- et R²¹ représente un groupe alkyle en C₁-C₃,
R²⁰ représentant
un atome d'hydrogène,
un groupe alkyle, alcényle ou alcynyle à chaîne droite ou ramifiée ayant jusqu'à 10 atomes de carbone,
R²⁰ et R²¹ mais non les deux simultanément pouvant être le groupe méthyle,
ou l'un des groupements
-D-CₙF₂ₙ₊₁, D étant un groupe alkylène, alcénylène ou alcynylène à chaîne droite ou ramifiée ayant jusqu'à 8 atomes de carbone et n étant un nombre entier allant de 1 à 8,
D-aryle, D ayant la signification déjà indiquée et aryle représentant un radical phényle, 1- ou 2-naphtyle éventuellement une ou deux fois substitués ou un radical hétéroaryle,
-L-CH=CF-CₚF₂ₚ₊₁, L étant un groupe alkylène, alcénylène ou alcynylène à chaîne droite ou ramifiée ayant jusqu'à 7 atomes de carbone et p étant un nombre entier allant de 1 à 7,
dans les trois cas précédents un groupe méthylène dans D ou L pouvant être remplacé par un atome de soufre, un groupe sulfone ou sulfoxyde,
-D-O-(CH₂)_{q}-aryle, D et aryle ayant les significations déjà indiquées et q étant 0, 1, 2 ou 3,
-D-O-(CH₂)ᵣ-CₙF₂ₙ₊₁, D ayant les significations déjà indiquées et r représentant un nombre entier allant de 1 à 5,
en outre dans tous les cas précédents concernés R²¹ pouvant former conjointement avec D et avec inclusion de l'atome d'azote un cycle pyrrolidine alors substitué en position 2 ou 3,
ou
R²⁰ et R²¹ forment, avec l'atome d'azote auquel il sont fixés, un hétérocycle saturé ou insaturé ayant 5 ou 6 chaînons formant le cycle, qui contient éventuellement un ou deux autres hétéroatomes choisis parmi les atomes d'azote, d'oxygène et de soufre, et est éventuellement substitué, et
R^{17a} représente en position α ou β un atome d'hydrogène, un groupe alkyle en C₁-C₅, un groupe alcényle en C₂-C₅ ou un groupe alcynyle en C₂-C₅ ou un groupe trifluorométhyle,
Ou, conjointement avec le radical OR^{17b}, un atome d'oxygène en fonction céto, et
R^{17b} représente un atome d' hydrogène ou un radical de formule partielle R^{17'}-C(O)-, R^{17'} représentant un atome d'hydrogène ou un radical hydrocarboné ayant jusqu'à 8 atomes de carbone.

2. Estratriènes substitués en 11β selon la revendication 1, dans lesquels R³ est un atome d'hydrogène.

3. Estratriènes substitués en 11β selon la revendication 1, dans lesquels R³ est un radical benzoyle.

4. Estratriènes substitués en 11β selon la revendication 1, dans lesquels R^{17b} est un atome d'hydrogène.

5. Estratriènes substitués en 11β selon la revendication 1, dans lesquels R¹¹ est choisi dans l'ensemble des chaînes latérales suivantes :
-(CH₂)₅N(CH₃)-(CH₂)₃-S-(CH₂)₃C₂F₅
-(CH₂)₅NH-(CH₂)₃-S-(CH₂)₃C₂F₅
-(CH₂)₅N(CH₃)-(CH₂)₃-S-CH₂-2-pyridyle
-(CH₂)₅N(CH₃)-(CH₂)₃-SO-CH₂-2-pyridyle
-(CH₂)₅N(CH₃)-(CH₂)₃-S-CH₂-p-CF₃-phényle
-(CH₂)₅N(CH₃)-(CH₂)₃-SO-CH₂-p-CF₃-phényle
-(CH₂)₅-[2-pyrrolidin-1-yl]-CH₂-S-p-CF₃-phényle
-(CH₂)₅-[2-pyrrolidin-1-yl]-CH₂-SO-p-CF₃-phényle
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅N(CH₃)CH₂CH=CF-C₂F₅
-(CH₂)₅N(CH₃)CH₂CH=CF-C₃F₇
-(CH₂)₅N(CH₃)CH₂CH=CF-C₅F₁₁
-(CH₂)₅N(CH₃)CH₂CH=CF-C₇F₁₅
-(CH₂)₅-1-pyrrolidinyle
-(CH₂)₅N(CH₃)(CH₂)₃Ophényle
-(CH₂)₅N(CH₃)(CH₂)₃Obenzyle
-(CH₂)₅N(CH₃)(CH₂)₃O(CH₂)₃C₂F₅
-(CH₂)₅N(CH₃)(CH₂)₃CH(CH₃)₂
-(CH₂)₅N(CH₃)(CH₂)₃-pyridyle
-(CH₂)₅N(CH₃)(CH₂)₃-phényle
-(CH₂)₅N(CH₃)(CH₂)₂-p-tolyle
-(CH₂)₅N(CH₃)(CH2)₂-p-éthoxyphényle
-(CH₂)₅N(CH₃)(CH₂)₃-p-tolyle
-(CH₂)₅N(CH₃)(CH₂)₃-p-chlorophényle
-(CH₂)₅N(CH₃)(CH₂)₃-O-CH₂-phényle
-(CH₂)₅N(CH₃)(CH₂)₂-O-p-Br-phényle
-(CH₂)₅N(CH₃)(CH₂)₂-O-p-CF₃-phényle

6. Estratriènes substitués en 11β de formule générale I, à savoir
11β-[5-(méthyl{3-[(4,4,5,5,5-pentafluorpentyl)-sulfanyl]propyl}amino)pentyl]estra-1,3,5(10)-triène-3,17β-diol
11β-(5-[3-[(4,4,5,5,5-pentafluoropentyl)sulfanyl]-propylamino]pentyl)estra-1,3,5(10)-triène-3,17β-diol
11β-[5-(méthyl{3-[(2-pyridylméthyl)sulfanyl]-propyl}amino)pentyl]estra-1,3,5(10)-triène-3,17β-diol
11β-[5-(méthyl{3-[(2-pyridylméthyl)sulfinyl]-propyl}amino)pentyl)estra-1,3,5(10)-triène-3,17β-diol
11β-[5-(méthyl{3-[4-(trifluorométhyl)-benzylsulfanyl]propyl}amino)pentyl]estra-1,3,5(10)-triène-3,17β-diol
11β-[5-(méthyl{3-[4-(trifluorométhyl)-benzylsulfinyl]propyl}amino)pentyl]estra-1,3,5(10)-triène-3,17β-diol)
11β-{5-[(2S)-2-{[4-(trifluorométhyl)phényl]-sulfanylméthyl}pyrrolidin-l-yl]pentyl}estra-1,3,5(10)-triène-3,17β-diol
11β-{5-[(2S)-2-{[4-(trifluorométhyl)phényl]-sulfinylméthyl}pyrrolidin-1-yl]pentyl}estra-1,3,5(10)-triène-3,17β-diol
11β-{5-[méthyl-(8,8,9,9,9-pentafluoro-nonyl)-amino]-pentyl}-estra-1,3,5(10)-triène-3,17β-diol
11β-{5-[méthyl-nonyl-amino]-pentyl}-estra-1,3,5(10)-triène-3,17β-diol
11β-{5-[méthyl-(9,9,10,10,10-pentafluoro-décyl)-amino]-pentyl}-estra-1,3,5(10)-triène-3,17(3-diol
11β-{6-[méthyl-(8,8,9,9,9-pentafluoro-nonyl)-amino]-hexyl}-estra-1,3,5(10)-triène-3,17β-diol
11β-{6-[méthyl-(9,9,10,10,10-pentafluoro-décyl)-amino]-hexyl}-estra-1,3,5(10)-triène-3,17β-diol
11β-(5-(méthyl-amino)-pentyl)-estra-1,3,5(10)-triène-3, 17β-diol
11β-(5-pyrrolidin-1-yl-pentyl)-estra-1,3,5(10)-triène-3,17β-diol
11β-{5-[méthyl-(4,4,5,5,5-pentafluoro-pentyl)-amino]-pentyl}-estra-1,3,5(10)-triène-3,17β-diol
11β-{5-[méthyl-(4,4,5,5,6,6,7,7,8,8,9,9,9-tridécafluoro-nonyl)-amino]-pentyl}-estra-1,3,5(10)-triène-3,17β-diol
11β-{5-[(4,4,5,5,6,6,7,7,8,8,9,9,10,10,11,11,11-heptadécafluoro-indécyl)-méthyl-amino]-pentyl}-estra-1,3,5(10)-triène-3,17β-diol
11β-{5-[méthyl-(3,3,4,4,5,5,6,6,6-nonafluoro-hexyl)-amino]-pentyl}-estra-1,3,5(10)-triène-3,17β-diol
11β-{5-[méthyl-(7,7,8,8,8-pentafluoro-octyl)-amino]-pentyl}-estra-1,3,5(10)-triène-3,17β-diol
11β-{6-[méthyl-(7,7,8,8,8,-pentafluoro-octyl)-amino]-hexyl}-estra-1,3,5(10)-triène-3,17β-diol
11β-{5-[méthyl-(7,7,8,8,9,9,10,10,10-nonafluoro-décyl)-amino]-pentyl}-estra-1,3,5(10)-triène-3,17β-diol
11β-{5-[méthyl-(7,7,8,8,9,9,10,10,11,11,12,12,12-tridécafluoro-dodécyl)-amino]-pentyl}-estra-1,3,5(10)-triène-3,17β-diol
11β-{5-[(7,7,8,8,9,9,10,10,11,11,12,12,13,13,14,14,14-heptadécafluoro-tétradécyl)-méthylamino]-pentyl}-estra-1,3,5(10)-triène-3,17β-diol
11β-{5-[(3,4,4,5,5,5-hexafluoro-pent-2-ényl)-méthyl-amino]-pentyl}-estra-1,3,5(10)-triène-3,17β-diol
11β-{5-[(3,4,4,5,5,6,6,7,7,8,8,8-dodécafluoro-oct-2-ényl)-méthyl-amino]-pentyl}-estra-1,3,5(10)-triène-3,17β-diol
11β-{5-[(3,4,4,5,5,6,6,7,7,8,8,9,9,10,10,10-hexadécafluoro-déc-2-ényl)-méthyl-amino]-pentyl}-estra-1,3,5(10)-triène-3,17β-diol
11β-{5-[méthyl-(3-phénoxy-propyl)-amino]-pentyl}-estra-1,3,5 (10)-triène-3,17β-diol
11β-{5-[(3-benzyloxy-propyl)-méthyl-amino]-pentyl}-estra-1,3,5(10)-triène-3,17β-diol
11β-{5-[N-méthyl-N-3-(4,4,5,5,5-pentafluoropentyloxy)-propylamino]-pentyl}-estra-1,3,5(10)-triène-3,17β-diol
11β-{5-[méthyl-(2-p-tolyl-éthyl)-amino]-pentyl}-estra-1,3,5(10)-triène-3,17(3-diol
11β-(5-{[2-(4-éthoxy-phényl)-éthyl]-méthyl-amino}-pentyl)-estra-1,3,5(10)-triène-3,17(3-diol
11(3-{5-[méthyl-(3-phényl-propyl)-amino]-pentyl}-estra-1,3,5(10)-triène-3,17(3-diol
11β-{5-[méthyl-(3-pyridin-3-yl-propyl)-amino]-pentyl}-estra-1,3,5(10)-triène-3,17β-diol
11β-{5-[méthyl-(3-p-tolyl-propyl)-amino]-pentyl}-estra-1,3,5(10)-triène-3,17β-diol
11β-(5-{[3-(4-chloro-phényl)-propyl]-méthyl-amino}-pentyl)-estra-1,3,5(10)-triène-3,17β-diol
11β-(5-{[3-(4-éthoxy-phényl)-propyl]-méthyl-aminol-pentyl)-estra-1,3,5(10)-triène-3,17β-diol
11β-{5-[méthyl-(4-méthyl-pentyl)-amino]-pentyl}-estra-1,3,5(10)-triène-3,17β-diol.

7. Utilisation des composés de formule générale I selon la revendication 1, pour la fabrication de médicaments.

8. Préparations pharmaceutiques contenant au moins un composé de formule générale I selon la revendication 1, ainsi qu'un véhicule pharmaceutiquement acceptable.
